# EUROPEAN PATENT SPECIFICATION

(11) **EP 1 381 357 B1**
(45) Date of publication and mention of the grant of the patent: **20.10.2010**
(21) Application number: 02704938.6
(22) Date of filing: 07.03.2002
(51) Int. Cl.: C07C 62/32, C07C 62/34, C07C 62/38, C07C 69/753, C07C 235/40, C07D 295/13, C07D 295/182, C07D 213/40, A61K 31/16, A61K 31/19, A61K 31/57, A61P 5/38, A61P 5/44, A61P 5/46

(54) **GLYCYRRHETINIC ACID DERIVATIVES AND THEIR USE FOR THE MANUFACTURE OF A MEDICAMENT TO INHIBIT 11BETA-HYDROXYSTEROID DEHYDROGENASE ACTIVITY**
GLYCYRRHETINSÄUREDERIVATE UND IHRE VERWENDUNG ZUR HERSTELLUNG EINES MEDIKAMENTS ZUR HEMMUNG DER 11-BETA-HYDROXYSTEROID DEHYDROGENASE
DERIVÉS DE L'ACIDE GLYCYRRHETINIQUE ET LEUR UTILISATION POUR L'OBTENTION D'UN MEDICAMENT DESTINÉ À L'INHIBITION DE LA 11 BETA- HYDROXYSTEROID DEHYDROGENASE

(30) Priority: 08.03.2001 GB 0105772
(43) Date of publication of application: 21.01.2004
(73) Proprietor: Sterix Limited, Slough, Berkshire SL1 3XE (GB)
(72) Inventor: POTTER, Barry, Victor, Lloyd, Sterix Limited, Slough Berkshire SL1 3XE (GB); PUROHIT, Atul, Sterix Limited, Slough Berkshire SL1 3XE (GB); REED, Michael, John, Sterix Limited, Slough Berkshire SL1 3XE (GB); VICKER, Nigel, Sterix Limited, Slough Berkshire SL1 3XE (GB)
(74) Representative: Alcock, David
(86) International application number: PCT/GB2002/001060
(87) International publication number: WO 2002/072084

(56) References cited:
- EP-A1- 0 272 478
- WO-A-97/07789
- GB-A- 1 570 394
- US-A- 4 173 648
- US-A- 4 448 788
- BÜHLER H ET AL: "Inhibition of rat renal 11 beta-hydroxysteroid dehydrogenase by steroidal compounds and triterpenoids;structure/function relationship." BIOCHIMICA ET BIOPHYSICA ACTA. NETHERLANDS 31 OCT 1991, vol. 1075, no. 3, 31 October 1991 (1991-10-31), pages 206-212, XP001080642 ISSN: 0006-3002
- AKAO T ET AL: "Inhibitory effects of glycyrrhetic acid derivatives on 11 beta- and 3 alpha-hydroxysteroid dehydrogenases of rat liver." CHEMICAL & PHARMACEUTICAL BULLETIN. JAPAN NOV 1992, vol. 40, no. 11, November 1992 (1992-11), pages 3021-3024, XP001080148 ISSN: 0009-2363
- STEWART P M ET AL: "11BETA-HYDROXYSTEROID DEHYDROGENASE" VITAMINS AND HORMONES, ACADEMIC PRESS, NEW YORK, NY,, US, vol. 57, 1999, pages 249-324, XP001004592 ISSN: 0083-6729
- DUAN H ET AL: "Triterpenoids from Tripterygium wilfordii" PHYTOCHEMISTRY, PERGAMON PRESS, GB, vol. 53, no. 7, 1 April 2000 (2000-04-01), pages 805-810, XP004291375 ISSN: 0031-9422
- SUN, DI-AN ET AL: "DNA Polymerase.beta. Inhibitors from Sandoricum koetjape" JOURNAL OF NATURAL PRODUCTS (1999), 62(8), 1110-1113 , XP002201254
- TAKAISHI Y ET AL: "Triterpenoid inhibitors of interleukin-1 secretion and tumour-promotion from Tripterygium wilfordii var. regelii" PHYTOCHEMISTRY, PERGAMON PRESS, GB, vol. 45, no. 5, July 1997 (1997-07), pages 969-974, XP004293236 ISSN: 0031-9422
- DATABASE CA [Online] CHEMICAL ABSTRACTS SERVICE, COLUMBUS, OHIO, US; KONDRATENKO, R. M. ET AL: "Synthesis and antiulcer activity of 3-O-acylated glycyrrhetic acid methylates" retrieved from STN Database accession no. 136:247713 XP002201255 & PHARMACEUTICAL CHEMISTRY JOURNAL (TRANSLATION OF KHIMIKO-FARMATSEVTICHESKII ZHURNAL) (2001), 35(5), 243-246 ,
- DATABASE CA [Online] CHEMICAL ABSTRACTS SERVICE, COLUMBUS, OHIO, US; KHAKSA, G. ET AL: "Antiinflammatory and antinociceptive activity of disodium glycyrrhetinic acid hemiphthalate" retrieved from STN Database accession no. 125:292578 XP002201256 & PLANTA MED. (1996), 62(4), 326-328 ,
- DATABASE CA [Online] CHEMICAL ABSTRACTS SERVICE, COLUMBUS, OHIO, US; YANO, SHINGO ET AL: "Antiulcer activities of glycyrrhetinic acid derivatives in experimental gastric lesion models" retrieved from STN Database accession no. 112:16107 XP002201257 & CHEM. PHARM. BULL. (1989), 37(9), 2500-4 ,
- DATABASE CA [Online] CHEMICAL ABSTRACTS SERVICE, COLUMBUS, OHIO, US; SHIBATA, S.: "Medicinal chemistry of triterpenoid saponins and sapogenins" retrieved from STN Database accession no. 95:73516 XP002201258 & PROC. ASIAN SYMP. MED. PLANTS SPICES, 4TH (1981), MEETING DATE 1980, VOLUME 1, 59-70. EDITOR(S): KUSAMRAN, KOSAN;POHMAKOTR, MANAT; REUTRAKUL, VICHAI. PUBLISHER: AKSORN CHAROEN - TAT PUBL. HOUSE, BANGKOK, THAILAND. ,
- PINDUCCIU, G.; SERRA, C.; CAGETTI, M.G.; COTTI, M.; DEIDDA, D.; PINZA, M.; POMPEI, R.: "Selective Antibacterial Activity of Triterpene Derivatives" MED. MICROBIOL. LETT., vol. 4, no. 2, 1995, pages 83-90, XP001098298
- PELLEGATA, R.; PINZA, M.; PIFFERI, G.; FARINA, C.: "A new reduction of the Enone-System of 18-beta-Glycyrrhetic Acid" ORGANIC PREPARATIONS AND PROCEDURES INT., vol. 31, no. 2, 1999, pages 181-187, XP001098252
- LATIF S A ET AL: "Selective inhibition of sheep kidney 11beta-hydroxysteroid dehydrogenase isoform 2 activity by 5alpha-reduced (but not 5beta) derivatives of adrenocorticosteroids" STEROIDS: STRUCTURE, FUNCTION, AND REGULATION, ELSEVIER SCIENCE PUBLISHERS, NEW YORK, NY, US, vol. 62, no. 2, 1 February 1997 (1997-02-01), pages 230-237, XP004056328 ISSN: 0039-128X
- PHILLIPPS G H ET AL: "WATER-SOLUBLE STEROIDAL ANAESTHETICS" JOURNAL OF STEROID BIOCHEMISTRY, PERGAMON PRESS PLC, GB, vol. 11, no. 1A, 1 July 1979 (1979-07-01), pages 79-86, XP000600717 ISSN: 0022-4731
- HULT M ET AL: "Selective inhibition of human type 1 11beta-hydroxysteroid dehydrogenase by synthetic steroids and xenobiotics" FEBS LETTERS, ELSEVIER SCIENCE PUBLISHERS, AMSTERDAM, NL, vol. 441, no. 1, 11 December 1998 (1998-12-11), pages 25-28, XP004258864 ISSN: 0014-5793
- WU P ET AL: "Effects of cholic acid on blood pressure and production of vascular aldosterone and corticosterone - Implications for the pathophysiology of hypertension" STEROIDS: STRUCTURE, FUNCTION, AND REGULATION, ELSEVIER SCIENCE PUBLISHERS, NEW YORK, NY, US, vol. 64, no. 4, April 1999 (1999-04), pages 291-295, XP004172206 ISSN: 0039-128X
- ALIBAEVA KH. A. ET AL: 'Triterpenoids. XXXI. Beckmann rearrangement of glycyrrhetic acid amides' IZVESTIYA AKADEMII NAUK KAZAKHSKOI SSR, SERIYA KHIMICHESKAYA vol. 25, no. 6, 1975, pages 39 - 42

## Description

The present invention relates to use of compounds to inhibit 11β-hydroxysteroid dehydrogenase (11β-HSD).

### Introduction

### The role of glucocorticoids

Glucocorticoids are synthesised in the adrenal cortex from cholesterol. The principle glucocorticoid in the human body is cortisol, this hormone is synthesised and secreted in response to the adrenocortictrophic hormone (ACTH) from the pituitary gland in a circadian, episodic manner, but the secretion of this hormone can also be stimulated by stress, exercise and infection. Cortisol circulates mainly bound to transcortin (cortisol binding protein) or albumin and only a small fraction is free (5-10%) for biological processes [1].

Cortisol has a wide range of physiological effects, including regulation of carbohydrate, protein and lipid metabolism, regulation of normal growth and development, influence on cognitive function, resistance to stress and mineralocorticoid activity. Cortisol works in the opposite direction compared to insulin meaning a stimulation of hepatic gluconeogenesis, inhibition of peripheral glucose uptake and increased blood glucose concentration. Glucocorticoids are also essential in the regulation of the immune response. When circulating at higher concentrations glucocorticoids are immunosuppressive and are used pharmacologically as anti-inflammatory agents.

Glucocorticoids like other steroid hormones are lipophilic and penetrate the cell membrane freely. Cortisol binds, primarily, to the intracellular glucocorticoid receptor (GR) that then acts as a transcription factor to induce the expression of glucocorticoid responsive genes, and as a result of that protein synthesis.

### The role of the 11β-HSD enzyme

The conversion of cortisol (F) to its inactive metabolite cortisone (E) by 11β-HSD was first described in the 1950's, however it was not until later that the biological importance for this conversion was suggested [2]. In 1983 Krozowski et al. showed that the mineralocorticoid receptor (MR) has equal binding affinities for glucocorticoids and mineralocorticoids [3]. Because the circulating concentration of cortisol is a 100 times higher than that of aldosterone and during times of stress or high activity even more, it was not clear how the MR remained mineralocorticoid specific and was not constantly occupied by glucocorticoids. Earlier Ulick et al. had described the hypertensive condition known as, "apparent mineralocorticoid excess" (AME), and observed that whilst secretion of aldosterone from the adrenals was in fact low the peripheral metabolism of cortisol was disrupted. These discoveries lead to the suggestion of a protective role for the enzymes. By converting cortisol to cortisone in mineralocorticoid dependent tissues 11β-HSD enzymes protects the MR from occupation by glucocorticoids and allows it to be mineralcorticoid specific. Aldosterone itself is protected from the enzyme by the presence of an aldehyde group at the C-18 position.

Congenital defects in the 11β-HSD enzyme results in over occupation of the MR by cortisol and hypertensive and hypokalemic symptoms seen in AME.

Localisation of the 11β-HSD showed that the enzyme and its activity is highly present in the MR dependent tissues, kidney and parotid. However in tissues where the MR is not mineralocorticoid specific and is normally occupied by glucocorticoids, 11β-HSD is not present in these tissues, for example in the heart and hippocampus [5]. This research also showed that inhibition of 11 β-HSD caused a loss of the aldosterone specificity of the MR in these mineralocorticoid dependent tissues.

It has been shown that two iso-enzymes of 11 β-HSD exist. Both are members of the *short chain alcohol dehydrogenase* (SCAD) superfamily which have been widely conserved throughout evolution. 11 β-HSD type 2 acts as a dehydrogenase to convert the secondary alcohol group at the C-11 position of cortisol to a secondary ketone, so producing the less active metabolite cortisone. 11 β-HSD type 1 is thought to act mainly in vivo as a reductase, that is in the opposite direction to type 2 [6] [see below]. 11 β-HSD type 1 and type 2 have only a 30% amino acid homology.

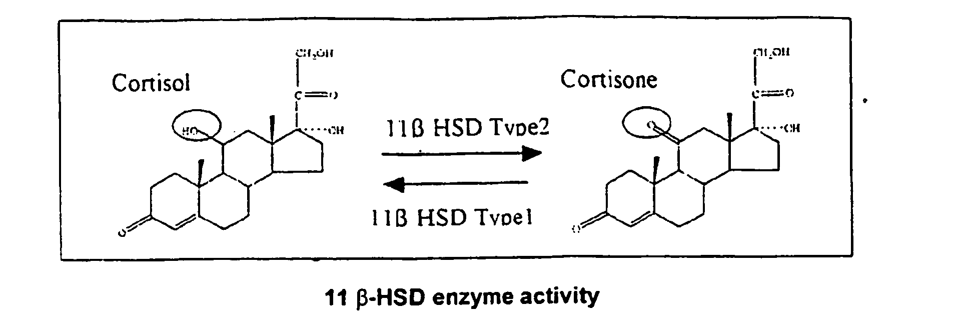

The intracellular activity of cortisol is dependent on the concentration of glucocorticoids and can be modified and independently controlled without involving the overall secretion and synthesis of the hormone.

### The role of 11β-HSD Type 1

The direction of 11 β-HSD type 1 reaction in vivo is generally accepted to be opposite to the dehydrogenation of type 2. In vivo homozygous mice with a disrupted type 1 gene are unable to convert cortisone to cortisol, giving further evidence for the reductive activity of the enzyme [7]. 11 β-HSD type 1 is expressed in many key glucocorticoid regulated tissues like the liver, pituitary, gonad, brain, adipose and adrenals ,however, the function of the enzyme in many of these tissues is poorly understood [8].

The concentration of cortisone in the body is higher than that of cortisol, cortisone also binds poorly to binding globulins, making cortisone many times more biologically available. Although cortisol is secreted by the adrenal cortex, there is a growing amount of evidence that the intracellular conversion of E to F may be an important mechanism in regulating the action of glucocorticoids [9].

It may be that 11 β-HSD type 1 allows certain tissues to convert cortisone to cortisol to increase local glucocorticoid activity and potentiate adaptive response and counteracting the type 2 activity that could result in a fall in active glucocorticoids [10]. Potentiation of the stress response would be especially important in the brain and high levels of 11 β-HSD type 1 are found around the hippocampus, further proving the role of the enzyme.

11 β-HSD type 1 also seems to play an important role in hepatocyte maturation [8]. Another emerging role of the 11 β-HSD type 1 enzyme is in the detoxification process of many non-steroidal carbonyl compounds, reduction of the carbonyl group of many toxic compounds is a common way to increase solubility and therefore increase their excretion. The 11 β-HSD type1 enzyme has recently been shown to be active in lung tissue [11]. Type 1 activity is not seen until after birth, therefore mothers who smoke during pregnancy expose their children to the harmful effects of tobacco before the child is able to metabolically detoxify this compound.

### The role of 11 β-HSD Type 2

As already stated earlier the 11 β-HSD type 2 converts cortisol to cortisone, thus protecting the MR in many key regulatory tissues of the body. The importance of protecting the MR from occupation by glucocorticoids is seen in patients with AME or liquorice intoxication. Defects or inactivity of the type 2 enzyme results in hypertensive syndromes and research has shown that patients with an hypertensive syndrome have an increased urinary excretion ratio of cortisol : cortisone. This along with a reported increase in the half life of radiolabelled cortisol suggests a reduction of 11 β-HSD type 2 activity [12].

### Rationale for the development of 11 β-HSD inhibitors

As said earlier cortisol opposes the action of insulin meaning a stimulation of hepatic gluconeogenesis, inhibition of peripheral glucose uptake and increased blood glucose concentration. The effects of cortisol appear to be enhanced in patients suffering from glucose intolerance or diabetes mellitus. Inhibition of the enzyme 11 β-HSD type 1 would increase glucose uptake and inhibit hepatic gluconeogenesis, giving a reduction in circulatory glucose levels. The development of a potent 11 β-HSD type 1 inhibitor could therefore have considerable therapeutic potential for conditions associated with elevated blood glucose levels.

An excess in glucocorticoids can result in neuronal dysfunctions and also impair cognitive functions. A specific 11 β-HSD type 1 inhibitor might be of some importance by reducing neuronal dysfunctions and the loss of cognitive functions associated with ageing, by blocking the conversion of cortisone to cortisol.

Glucocorticoids also have an important role in regulating part of the immune response [13]. Glucocorticoids can suppress the production of cytokines and regulate the receptor levels. They are also involved in determining whether T-helper (Th) lymphocytes progress into either Th1 or Th2 phenotype. These two different types of Th cells secrete a different profile of cytokines, Th2 is predominant in a glucocorticoid environment. By inhibiting 11 β-HSD type 1, Th1 cytokine response would be favoured. It is also possible to inhibit 11 β-HSD type 2, thus by inhibiting the inactivation of cortisol, it may be possible to potentiate the anti-inflammatory effects of glucocorticoids.

WO 97/07789 teaches the provision of a compound for inhibiting HSD Type 1 in vivo. Only one compound, carbenoxolone, is disclosed in this document. There is therefore a desire for additional compounds which may be used for the inhibition of HSD.

The present invention alleviates the problems of the prior art.

Aspects of the invention are defined in the appended claims.

In one aspect the present invention provides a compound of formula I or a salt thereof 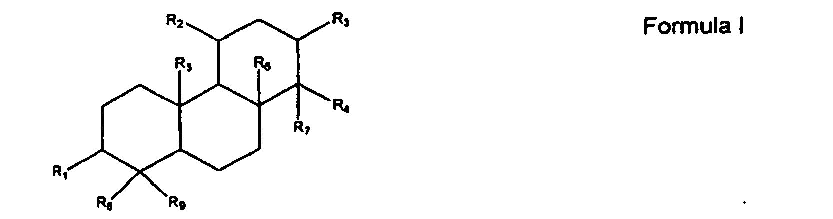 wherein R1 is O-CH₂-CH₂-Ph, O-CH₂-cyclohexyl, OH, O-alkyl, or O-aryl; and R2 is selected from H, =O, OH, hydrocarbyl, oxyhydrocarbyl, and halo; R5 to R9 are independently selected from H and hydrocarbyl
R3 and R4 together represent a group of formula II 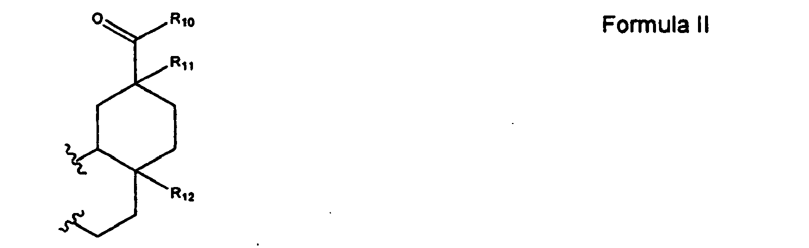 wherein R10 is selected from OH, hydrocarbyl, -NR₁₈R₁₉ and O-hydrocarbyl, R11 and R12 are independently selected from H and hydrocarbyl,
wherein when R1 is OH, R10 is -NR₁₈R₁₉;
wherein R₁₈ and R₁₉ are selected from the group consisting of H, CH₂Ph, CH(CH₃)COOC₂H₅, CH(CH₃)COOH, cyclopropane, 2-methylpyridine, 2-(4-ethylmorpholine) and CH₂(CH₂)₄OH;
and wherein the compound may contain one or more further degrees of unsaturation.

In one aspect the present invention provides use of a compound of the present invention in the manufacture of a medicament to inhibit 11β-HSD activity.

In one aspect the present invention provides use of a compound of the present invention in the manufacture of a medicament for use in the therapy of a condition or disease associated with 11β-HSD.

In one aspect the present invention provides use of a compound of the present invention in the manufacture of a medicament for use in the therapy of a condition or disease associated with adverse 11 β-HSD levels.

### SOME ADVANTAGES

One key advantage of the present invention is that the compounds of the present invention can act as 11 β-HSD inhibitors. The compounds may inhibit the interconversion of inactive 11-keto steroids with their active hydroxy equivalents. Thus present invention provides methods by which the conversion of the inactive to the active form may be controlled, and to useful therapeutic effects which may be obtained as a result of such control. More specifically, but not exclusively, the invention is concerned with interconversion between cortisone and cortisol in humans.

Another advantage of the compounds of the present invention is that they may be potent 11β-HSD inhibitors *in vivo.*

Some of the compounds of the present invention are also advantageous in that they may be orally active.

The present invention may provide for a medicament for one or more of (i) regulation of carbohydrate metabolism, (ii) regulation of protein metabolism, (iii) regulation of lipid metabolism, (iv) regulation of normal growth and/or development, (v) influence on cognitive function, (vi) resistance to stress and mineralocorticoid activity.

Some of the compounds of the present invention may also be useful for the preparation of medicaments for inhibiting hepatic gluconeogenesis. The present invention may also provide a medicament to relieve the effects of endogenous glucocorticoids in diabetes mellitus, obesity (including centripetal obesity), neuronal loss and/or the cognitive impairment of old age. Thus, in a further aspect, the invention provides the use of an inhibitor of 11β-HSD in the manufacture of a medicament for producing one or more therapeutic effects in a patient to whom the medicament is administered, said therapeutic effects selected from inhibition of hepatic gluconeogenesis, an increase in insulin sensitivity in adipose tissue and muscle, and the prevention of or reduction in neuronal loss/cognitive impairment due to glucocorticoid-potentiated neurotoxicity or neural dysfunction or damage.

From an alternative point of view, the invention provides a use of a compound of the invention in the preparation of a medicament for treatment of a human or animal patient suffering from a condition selected from the group consisting of: hepatic insulin resistance, adipose tissue insulin resistance, muscle insulin resistance, neuronal loss or dysfunction due to glucocorticoid potentiated neurotoxicity, and any combination of the aforementioned conditions.

### PREFERRED ASPECTS

The compound for use in the present invention is of formula I or a salt thereof 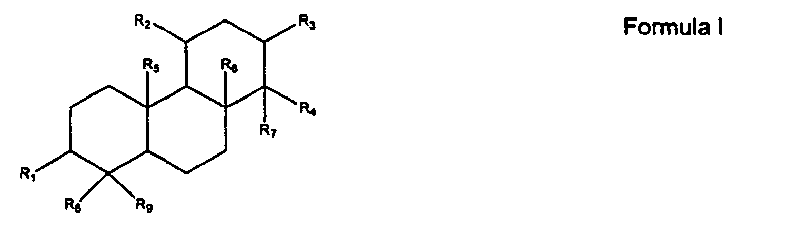 <A>: wherein R1 is O-CH₂-CH₂-Ph, O-CH₂-cyclohexyl, OH, O-alkyl, or O-aryl; and R2 is selected from H, =O, OH, hydrocarbyl, oxyhydrocarbyl, and halo; R5 to R9 are independently selected from H and hydrocarbyl R3 and R4 together represent a group of formula II 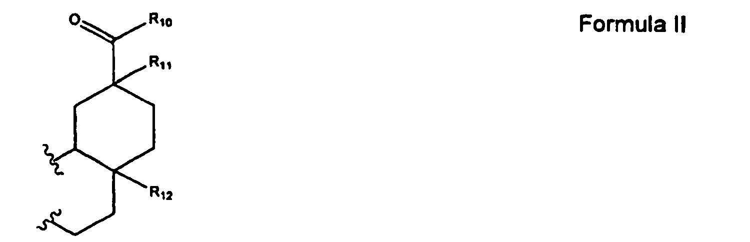 wherein R10 is selected from OH, hydrocarbyl, -NR₁₈R₁₉ and O-hydrocarbyl, R11 and R12 are independently selected from H and hydrocarbyl, wherein when R1 is OH, R10 is -NR₁₈R₁₉:
wherein R₁₈ and R₁₉ are selected from the group consisting of H, CH₂Ph, CH(CH₃)COOC₂H₅, CH(CH₃)COOH, cyclopropane, 2-methylpyridine, 2-(4-ethylmorpholine) and CH₂(CH₂)₄OH;
and wherein the compound may contain one or more further degrees of unsaturation.

The compound of or for use in the present invention may be substituted with additional substituents to those specifically recited in the general formulae of the present specification or may contain one or more further bonds/degrees of unsaturation.

The term "hydrocarbyl group" as used herein means a group comprising at least C and H and may optionally comprise one or more other suitable substituents. Examples of such substituents may include halo, alkoxy, nitro, an alkyl group, a cyclic group etc. In addition to the possibility of the substituents being a cyclic group, a combination of substituents may form a cyclic group. If the hydrocarbyl group comprises more than one C then those carbons need not necessarily be linked to each other. For example, at least two of the carbons may be linked via a suitable element or group. Thus, the hydrocarbyl group may contain hetero atoms. Suitable hetero atoms will be apparent to those skilled in the art and include, for instance, sulphur, nitrogen and oxygen. A nonlimiting example of a hydrocarbyl group is an acyl group.

A typical hydrocarbyl group is a hydrocarbon group. Here the term "hydrocarbon" means any one of an alkyl group, an alkenyl group, an alkynyl group, which groups may be linear, branched or cyclic, or an aryl group. The term hydrocarbon also includes those groups but wherein they have been optionally substituted. If the hydrocarbon is a branched structure having substituent(s) thereon, then the substitution may be on either the hydrocarbon backbone or on the branch; alternatively the substitutions may be on the hydrocarbon backbone and on the branch.

Typical hydrocarbyl groups are C₁-C₁₀ hydrocarbyl, C₁-C₅ hydrocarbyl or C₁-C₃ hydrocarbyl.

Typical hydrocarbon groups are C₁-C₁₀ hydrocarbon, C₁-C₅ hydrocarbon, C₁-C₃ hydrocarbon, alkyl groups, C₁-C₁₀ alkyl, C₁-C₅ alkyl and C₁-C₃ alkyl.

The hydrocarbyl/hydrocarbon/alkyl may be straight chain or branched and/or may be saturated or unsaturated.

The term "oxyhydrocarbyl" group as used herein means a group comprising at least C, H and O and may optionally comprise one or more other suitable substituents. Examples of such substituents may include halo-, alkoxy-, nitro-, an alkyl group, a cyclic group etc.

In addition to the possibility of the substituents being a cyclic group, a combination of substituents may form a cyclic group. If the oxyhydrocarbyl group comprises more than one C then those carbons need not necessarily be linked to each other. For example, at least two of the carbons may be linked *via* a suitable element or group. Thus, the oxyhydrocarbyl group may contain hetero atoms. Suitable hetero atoms will be apparent to those skilled in the art and include, for instance, sulphur and nitrogen.

In one embodiment of the present invention, the oxyhydrocarbyl group is a oxyhydrocarbon group.

Here the term "oxyhydrocarbon" means any one of an alkoxy group, an oxyalkenyl group, an oxyalkynyl group, which groups may be linear, branched or cyclic, or an oxyaryl group. The term oxyhydrocarbon also includes those groups but wherein they have been optionally substituted. If the oxyhydrocarbon is a branched structure having substituent(s) thereon, then the substitution may be on either the hydrocarbon backbone or on the branch; alternatively the substitutions may be on the hydrocarbon backbone and on the branch.

Typically, the oxyhydrocarbyl group is of the formula C₁₋₆O (such as a C₁₋₃O).

In one preferred aspect R1 is selected from =O, OH, O-aryl, O-acyl and O-alkyl.

In one preferred aspect R1 is O-CH₂-CH₂-Ph.

In one preferred aspect R1 is O-Me, O-Et or O-CH₂-cyclohexyl.

In one preferred aspect R2 is selected from H, =O, OH, O-alkylaryl, and halo.

In one preferred aspect R2 is selected from H, =O, OH, O-CH₂-Ph and F.

In one preferred aspect R2 is =O or OH.

In one preferred aspect R3 and R4 together represent a group of formula IV 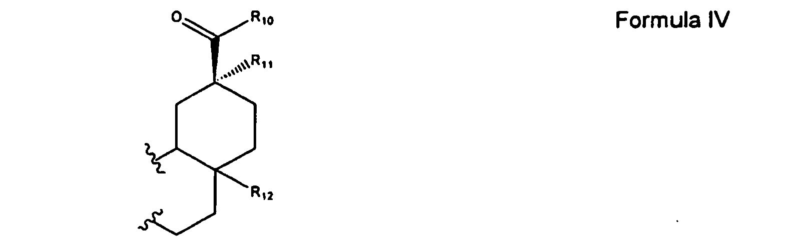 wherein R10, R11 and R12 are as defined above.

In one preferred aspect R3 and R4 together represent a group of formula V 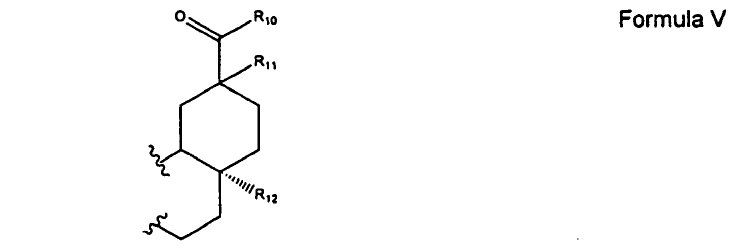 wherein R10, R11 and R12 are as defined above.

In the combination of these two preferred aspects R3 and R4 together represent a group of formula VI

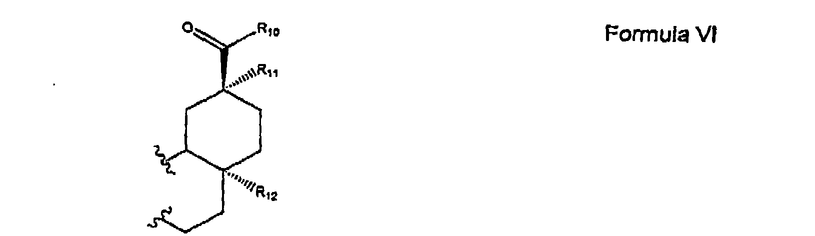

R10 of the compounds of the present invention is selected from OH, hydrocarbyl, -NR₁₈R₁₉ and O-hydrocarbyl. It will be appreciated that hydrocarbyl includes hydrocarbyl groups containing hetero atoms linking two carbons or linking one carbon to the compound of the invention. Thus hydrocarbyl incorporates for example N-hydrocarbyl and O-hydrocarbyl.

R₁₈ and R₁₉ are independently selected from H, CH₂Ph, CH(CH₃)COOC₂H₅, CH(CH₃)COOH, cyclopropane, 2-methylpyridine, 2-(4-ethylmorpholine), CH₂(CH₂)₄OH.

In one preferred aspect R10 is selected from OH and OMe.

In one preferred aspect R11 is Me.

In one preferred aspect R12 is Me.

In one preferred aspect R5 is Me.

In one preferred aspect R6 is Me or H.

In one preferred aspect R7 is Me.

In one preferred aspect R8 is H, Me or a bond with the carbon common with the adjacent ring.

In one preferred aspect R9 is H or Me.

In one aspect the present invention provides a use in the manufacture of a medicament for use in the therapy of a condition or disease associated with 11β-hydroxysteroid dehydrogenase wherein the compound is a novel compound of formula I or a salt thereof 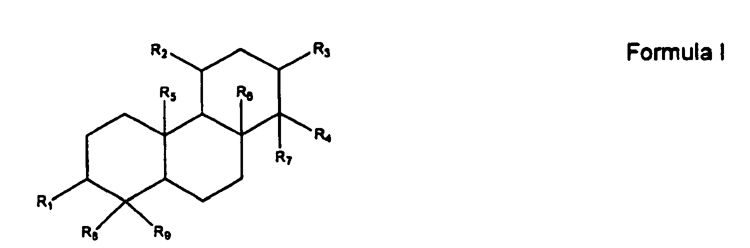 wherein R1 is O-CH₂-CH₂-Ph, O-CH₂-cyclohexyl, OH, O-alkyl, or O-aryl; and R2 is selected from H, =O, OH, hydrocarbyl, oxyhydrocarbyl, and halo; R5 to R9 are independently selected from H and hydrocarbyl
R3 and R4 together represent a group of formula II 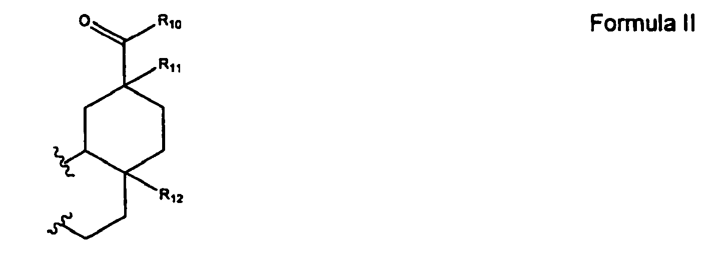 wherein R10 is selected from OH, hydrocarbyl, -NR₁₈R₁₉and O-hydrocarbyl, R11 and R12 are independently selected from H and hydrocarbyl,
wherein when R1 is OH, R10 is -NR₁₈R_{19;}
wherein R₁₈ and R₁₉ are selected from the group consisting of H, CH₂Ph, CH(CH₃)COOC₂H₅, CH(CH₃)COOH, cyclopropane, 2-methylpyridine, 2-(4-ethylmorpholine) and CH₂(CH₂)₄OH;
and wherein the compound may contain one or more further degrees of unsaturation.

In a further aspect the present invention provides a compound of formula I or a salt thereof 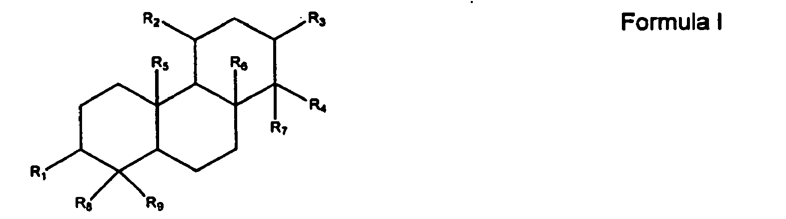 wherein R1 is OH, O-alkyl, or O-aryl
and R2 is selected from H, =O, OH, hydrocarbyl, oxyhydrocarbyl, and halo; R5 to R9 are independently selected from H and hydrocarbyl
R3 and R4 together represent a group of formula II 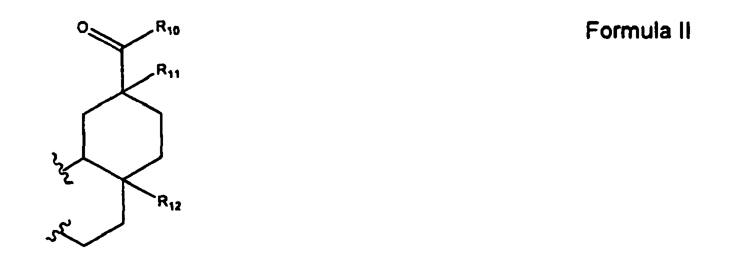 wherein R10 is selected from OH, hydrocarbyl, -NR₁₈R₁₉ and O-hydrocarbyl, R11 and R12 are independently selected from H and hydrocarbyl,
wherein when R1 is OH, R10 is -NR₁₈R₁₉.

In a further aspect the present invention provides a compound of formula I or a salt thereof 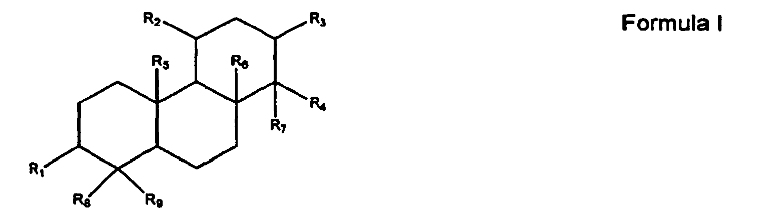 wherein R1 is O-alkyl or O-aryl
and R2 is selected from H, =O, OH, hydrocarbyl, oxyhydrocarbyl, and halo; R5 to R9 are independently selected from H and hydrocarbyl
R3 and R4 together represent a group of formula II 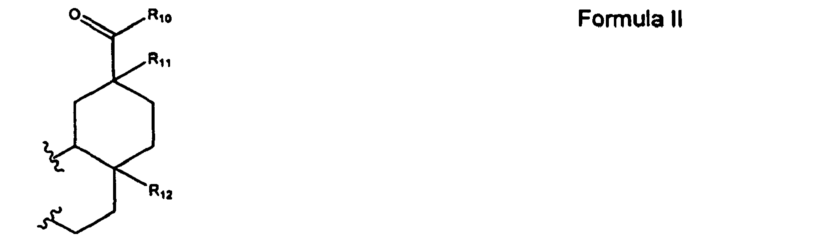 wherein R10 is selected from OH, hydrocarbyl, -NR₁₈R₁₉ and O-hydrocarbyl, R11 and R12 are independently selected from H and hydrocarbyl.

In these aspects wherein a novel compound is provided, preferably R1 is O-CH₂-CH₂-Ph; and/or R1 is O-Me, O-Et or O-CH₂-cyclohexyl; and/or R2 is O-CH₂-Ph.

In a further aspect the present invention provides a pharmaceutical composition comprising a novel compound as described herein optionally admixed with a pharmaceutically acceptable carrier, diluent, excipient or adjuvant.

In a further aspect the present invention provides a novel compound as described herein for use in medicine.

### THERAPY

The compounds of the present invention may be used in the preparation of therapeutic agents - i.e. in therapy applications.

The term "therapy" includes curative effects, alleviation effects, and prophylactic effects.

The therapy may be on humans or animals.

### PHARMACEUTICAL COMPOSITIONS

In one aspect, the present invention provides a pharmaceutical composition, which comprises a compound according to the present invention and optionally a pharmaceutically acceptable carrier, diluent or excipient (including combinations thereof).

The pharmaceutical compositions may be for human or animal usage in human and veterinary medicine and will typically comprise any one or more of a pharmaceutically acceptable diluent, carrier, or excipient. Acceptable carriers or diluents for therapeutic use are well known in the pharmaceutical art, and are described, for example, in Remington's Pharmaceutical Sciences, Mack Publishing Co. (A.R.Gennaro edit. 1985). The choice of pharmaceutical carrier, excipient or diluent can be selected with regard to the intended route of administration and standard pharmaceutical practice. The pharmaceutical compositions may comprise as - or in addition to - the carrier, excipient or diluent any suitable binder(s), lubricant(s), suspending agent(s), coating agent(s), solubilising agent(s).

Preservatives, stabilisers, dyes and even flavouring agents may be provided in the pharmaceutical composition. Examples of preservatives include sodium benzoate, sorbic acid and esters of p-hydroxybenzoic acid. Antioxidants and suspending agents may be also used.

There may be different composition/formulation requirements dependent on the different delivery systems. By way of example, the pharmaceutical composition of the present invention may be formulated to be delivered using a mini-pump or by a mucosal route, for example, as a nasal spray or aerosol for inhalation or ingestable solution, or parenterally in which the composition is formulated by an injectable form, for delivery, by, for example, an intravenous, intramuscular or subcutaneous route. Alternatively, the formulation may be designed to be delivered by both routes.

Where the agent is to be delivered mucosally through the gastrointestinal mucosa, it should be able to remain stable during transit though the gastrointestinal tract; for example, it should be resistant to proteolytic degradation, stable at acid pH and resistant to the detergent effects of bile.

Where appropriate, the pharmaceutical compositions can be administered by inhalation, in the form of a suppository or pessary, topically in the form of a lotion, solution, cream, ointment or dusting powder, by use of a skin patch, orally in the form of tablets containing excipients such as starch or lactose, or in capsules or ovules either alone or in admixture with excipients, or in the form of elixirs, solutions or suspensions containing flavouring or colouring agents, or they can be injected parenterally, for example intravenously, intramuscularly or subcutaneously. For parenteral administration, the compositions may be best used in the form of a sterile aqueous solution which may contain other substances, for example enough salts or monosaccharides to make the solution isotonic with blood. For buccal or sublingual administration the compositions may be administered in the form of tablets or lozenges which can be formulated in a conventional manner.

### COMBINATION PHARMACEUTICAL

The compound of the present invention may be used in combination with one or more other active agents, such as one or more other pharmaceutically active agents.

By way of example, the compounds of the present invention may be used in combination with other 11β-HSD inhibitors.

### ADMINISTRATION

Typically, a physician will determine the actual dosage which will be most suitable for an individual subject and it will vary with the age, weight and response of the particular patient. The dosages below are exemplary of the average case. There can, of course, be individual instances where higher or lower dosage ranges are merited.

The compositions of the present invention may be administered by direct injection. The composition may be formulated for parenteral, mucosal, intramuscular, intravenous, subcutaneous, intraocular or transdermal administration. Depending upon the need, the agent may be administered at a dose of from 0.01 to 30 mg/kg body weight, such as from 0.1 to 10 mg/kg, more preferably from 0.1 to 1 mg/kg body weight.

By way of further example, the agents of the present invention may be administered in accordance with a regimen of 1 to 4 times per day, preferably once or twice per day. The specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy.

Aside from the typical modes of delivery - indicated above - the term "administered" also includes delivery by techniques such as lipid mediated transfection, liposomes, immunoliposomes, lipofectin, cationic facial amphiphiles (CFAs) and combinations thereof. The routes for such delivery mechanisms include but are not limited to mucosal, nasal, oral, parenteral, gastrointestinal, topical, or sublingual routes.

The term "administered" includes but is not limited to delivery by a mucosal route, for example, as a nasal spray or aerosol for inhalation or as an ingestable solution; a parenteral route where delivery is by an injectable form, such as, for example, an intravenous, intramuscular or subcutaneous route.

Thus, for pharmaceutical administration, the 11β-HSD inhibitors of the present invention can be formulated in any suitable manner utilising conventional pharmaceutical formulating techniques and pharmaceutical carriers, adjuvants, excipients, diluents etc. and usually for parenteral administration. Approximate effective dose rates may be in the range from 1 to 1000 mg/day, such as from 10 to 900 mg/day or even from 100 to 800 mg/day depending on the individual activities of the compounds in question and for a patient of average (70Kg) bodyweight. More usual dosage rates for the preferred and more active compounds will be in the range 200 to 800 mg/day, more preferably, 200 to 500 mg/day, most preferably from 200 to 250 mg/day. They may be given in single dose regimes, split dose regimes and/or in multiple dose regimes lasting over several days. For oral administration they may be formulated in tablets, capsules, solution or suspension containing from 100 to 500 mg of compound per unit dose. Alternatively and preferably the compounds will be formulated for parenteral administration in a suitable parenterally administrable carrier and providing single daily dosage rates in the range 200 to 800 mg, preferably 200 to 500, more preferably 200 to 250 mg. Such effective daily doses will, however, vary depending on inherent activity of the active ingredient and on the bodyweight of the patient, such variations being within the skill and judgement of the physician.

The compounds of the present invention may be useful in the manufacture of a medicament for revealing an endogenous glucocorticoid-like effect.

### OTHER THERAPIES

It is also to be understood that the compound/composition of the present invention may have other important medical implications.

For example, the compound or composition of the present invention may be useful in the preparation of medicaments for the treatment of the disorders listed in WO-A-9W52890 - *viz*:

In addition, or in the alternative, the compound or composition of the present invention may be useful in the preparation of medicaments for the treatment of the disorders listed in WO-A-98/05635. For ease of reference, part of that list is now provided: cancer, inflammation or inflammatory disease, dermatological disorders, fever, cardiovascular effects, haemorrhage, coagulation and acute phase response, cachexia, anorexia, acute infection. HIV infection, shock states, graft-versus-host reactions, autoimmune disease, reperfusion injury, meningitis, migraine and aspirin-dependent anti-thrombosis; tumour growth, invasion and spread, angiogenesis, metastases, malignant, ascites and malignant pleural effusion; cerebral ischaemia, ischaemic heart disease, osteoarthritis, rheumatoid arthritis, osteoporosis, asthma, multiple sclerosis, neurodegeneration, Alzheimers disease, atherosclerosis, stroke, vasculitis, Crohn's disease and ulcerative colitis; periodontitis, gingivitis; psoriasis, atopic dermatitis, chronic ulcers, epidermolysis bullosa; corneal ulceration, retinopathy and surgical wound healing; rhinitis, allergic conjunctivitis, eczema, anaphylaxis; restenosis, congestive heart failure, endometriosis, atherosclerosis or endosclerosis.

In addition, or in the alternative, the compound or composition of the present invention may be useful in the preparation of medicaments for the treatment of disorders listed in WO-A-98107859. For ease of reference, part of that list is now provided: cytokine and cell proliferation/differentiation activity; immunosuppressant or immunostimulant activity (e.g. for treating immune deficiency, including infection with human immune deficiency virus; regulation of lymphocyte growth; treating cancer and many autoimmune diseases, and to prevent transplant rejection or induce tumour immunity); regulation of haematopoiesis, e.g. treatment of myeloid or lymphoid diseases; promoting growth of bone, cartilage, tendon, ligament and nerve tissue, e.g- for healing wounds, treatment of bums, ulcers and periodontal disease and neurodegeneration; inhibition or activation of follicle-stimulating hormone (modulation of fertility); chemotactic/chemokinetic activity (e.g. for mobilising specific cell types to sites of injury or infection); haemostatic and thrombolytic activity (e.g. for treating haemophilia and stroke); antiinflammatory activity (for treating e.g. septic shock or Crohn's disease); as antimicrobials; modulators of e.g. metabolism or behaviour, as analgesics; treating specific deficiency disorders; in treatment of e.g. psoriasis, in human or veterinary medicine.

In addition, or in the alternative, the composition of the present invention may be useful in the preparation of medicaments for treatment of disorders listed in WO-A-98/09985. For ease of reference, part of that list is now provided: macrophage inhibitory and/or T cell inhibitory activity and thus, anti-inflammatory activity; anti-immune activity, i.e. inhibitory effects against a cellular and/or humoral immune response, including a response not associated with inflammation; inhibit the ability of macrophages and T cells to adhere to extracellular matrix components and fibronectin, as well as up-regulated fas receptor expression in T cells; inhibit unwanted immune reaction and inflammation including arthritis, including rheumatoid arthritis, inflammation associated with hypersensitivity, allergic reactions, asthma, systemic lupus erythematosus, collagen diseases and other autoimmune diseases, inflammation associated with atherosclerosis, arteriosclerosis, atherosclerotic heart disease, reperfusion injury, cardiac arrest, myocardial infarction, vascular inflammatory disorders, respiratory distress syndrome or other cardiopulmonary diseases, inflammation associated with peptic ulcer, ulcerative colitis and other diseases of the gastrointestinal tract, hepatic fibrosis, liver cirrhosis or other hepatic diseases, thyroiditis or other glandular diseases, glomerulonephritis or other renal and urologic diseases, otitis or other oto-rhino-laryngological diseases, dermatitis or other dermal diseases, periodontal diseases or other dental diseases, orchitis or epididimo-orchitis, infertility, orchidal trauma or other immune-related testicular diseases, placental dysfunction, placental insufficiency, habitual abortion, eclampsia, pre-eclampsia and other immune and/or inflammatory-related gynaecological diseases, posterior uveitis, intermediate uveitis, anterior uveitis, conjunctivitis, chorioretinitis, uveoretinitis, optic neuritis, intraocular inflammation, e.g. retinitis or cystoid macular oedema, sympathetic ophthalmia, scleritis, retinitis pigmentosa, immune and inflammatory components of degenerative fondus disease, inflammatory components of ocular trauma, ocular inflammation caused by infection, proliferative vitreo-retinopathies, acute ischaemic optic neuropathy, excessive scarring, e.g. following glaucoma filtration operation, immune and/or inflammation reaction against ocular implants and other immune and inflammatory-related ophthalmic diseases, inflammation associated with autoimmune diseases or conditions or disorders where, both in the central nervous system (CNS) or in any other organ, immune and/or inflammation suppression would be beneficial, Parkinson's disease, complication and/or side effects from treatment of Parkinson's disease, AIDS-related dementia complex HIV-related encephalopathy, Devic's disease, Sydenham chorea, Alzheimer's disease and other degenerative diseases, conditions or disorders of the CNS, inflammatory components of stokes, post-polio syndrome, immune and inflammatory components of psychiatric disorders, myelitis, encephalitis, subacute sclerosing pan-encephalitis, encephalomyelitis, acute neuropathy, subacute neuropathy, chronic neuropathy, Guillaim-Barre syndrome, Sydenham chora, myasthenia gravis, pseudo-tumour cerebri, Down's Syndrome, Huntington's disease, amyotrophic lateral sclerosis, inflammatory components of CNS compression or CNS trauma or infections of the CNS, inflammatory components of muscular atrophies and dystrophies, and immune and inflammatory related diseases, conditions or disorders of the central and peripheral nervous systems, post-traumatic inflammation, septic shock, infectious diseases, inflammatory complications or side effects of surgery, bone marrow transplantation or other transplantation complications and/or side effects, inflammatory and/or immune complications and side effects of gene therapy, e.g. due to infection with a viral carrier, or inflammation associated with AIDS, to suppress or inhibit a humoral and/or cellular immune response, to treat or ameliorate monocyte or leukocyte proliferative diseases, e.g. leukaemia, by reducing the amount of monocytes or lymphocytes, for the prevention and/or treatment of graft rejection in cases of transplantation of natural or artificial cells, tissue and organs such as cornea, bone marrow, organs, lenses, pacemakers, natural or artificial skin tissue.

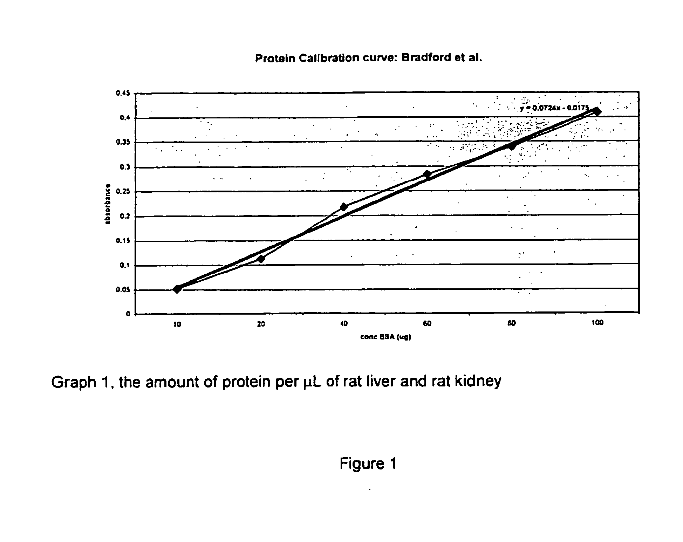

The present invention will now be described in further detail by way of example only with reference to the accompanying figures in which:-
Figure 1 shows a graph;

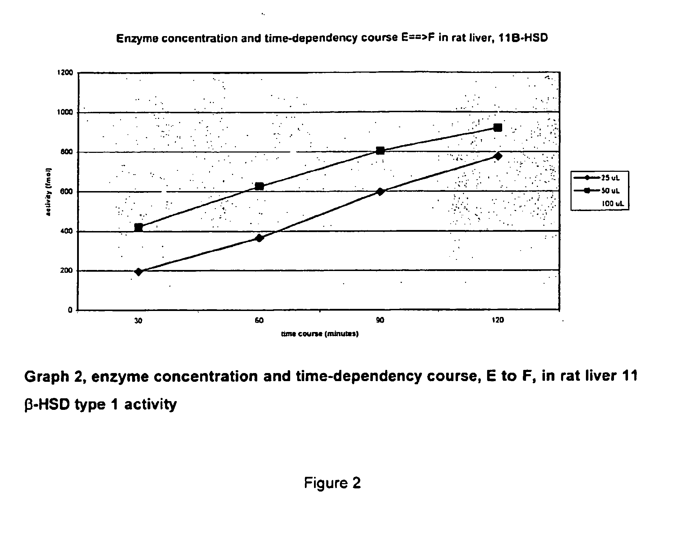

Figure 2 shows a graph;

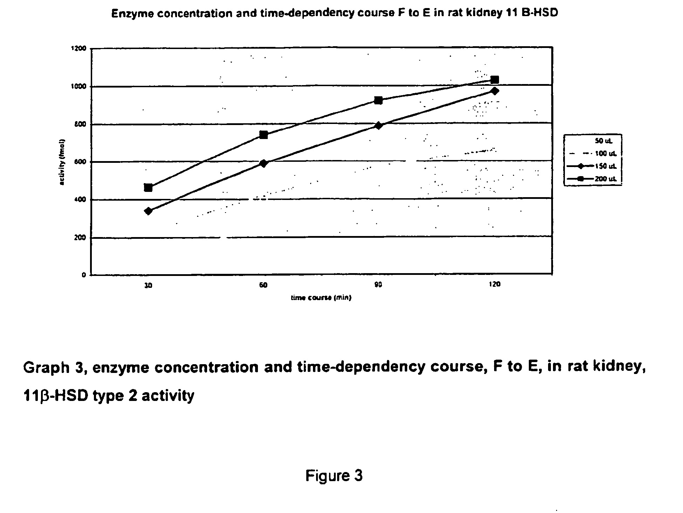

Figure 3 shows a graph;

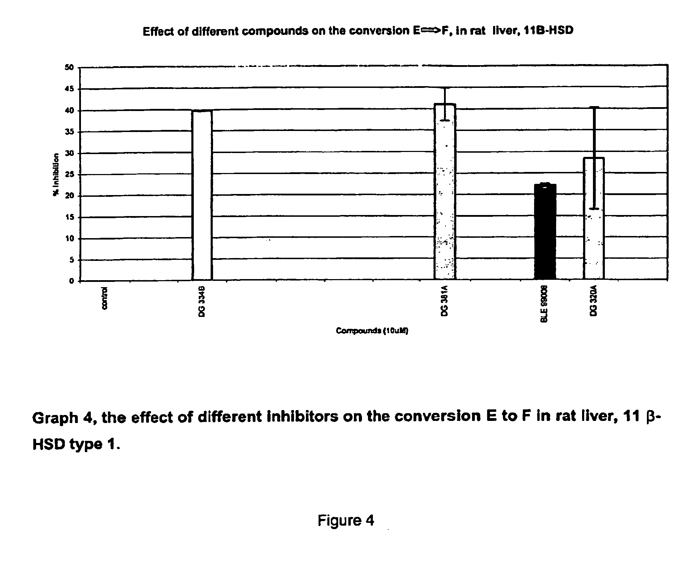

Figure 4 shows a graph;

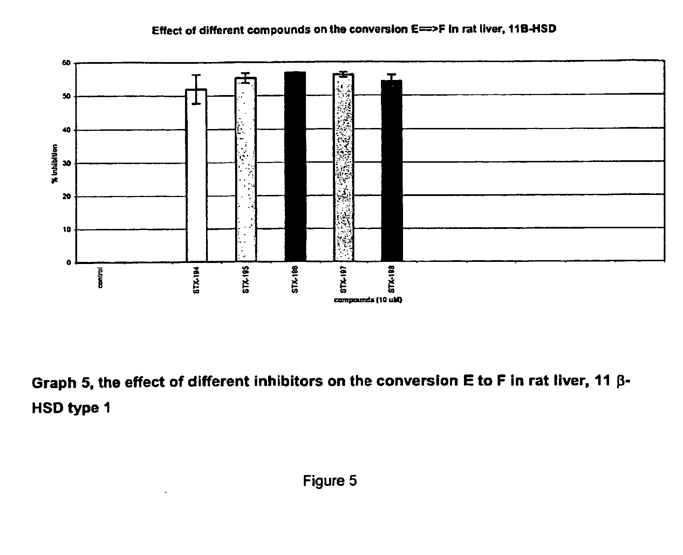

Figure 5 shows a graph;

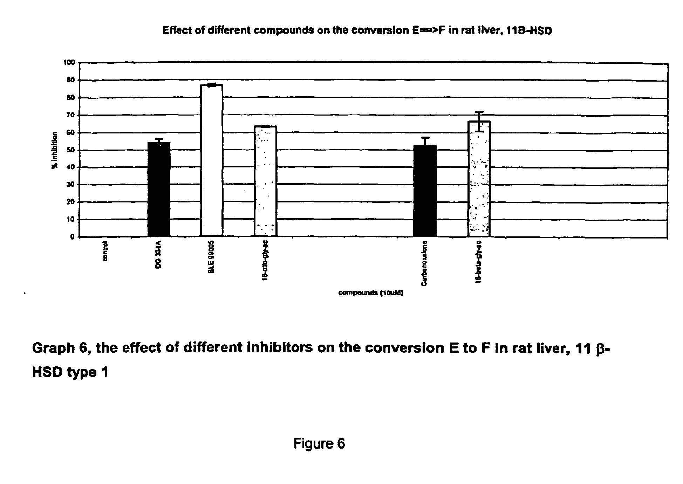

Figure 6 shows a graph;

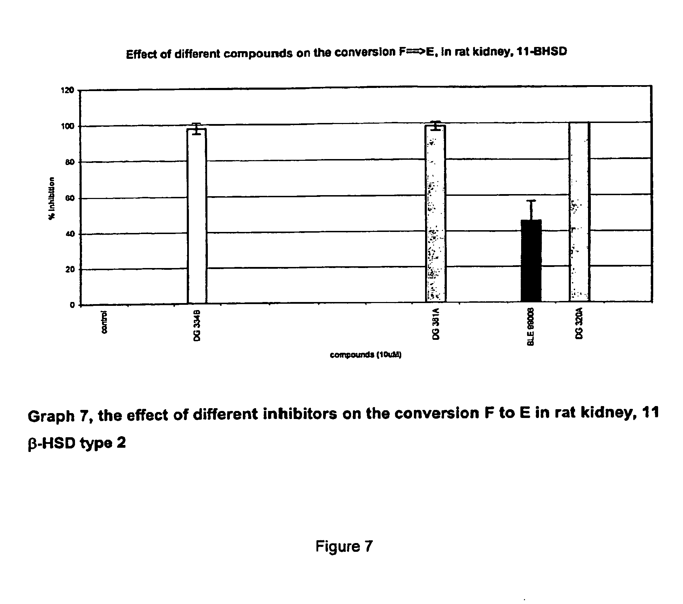

Figure 7 shows a graph;

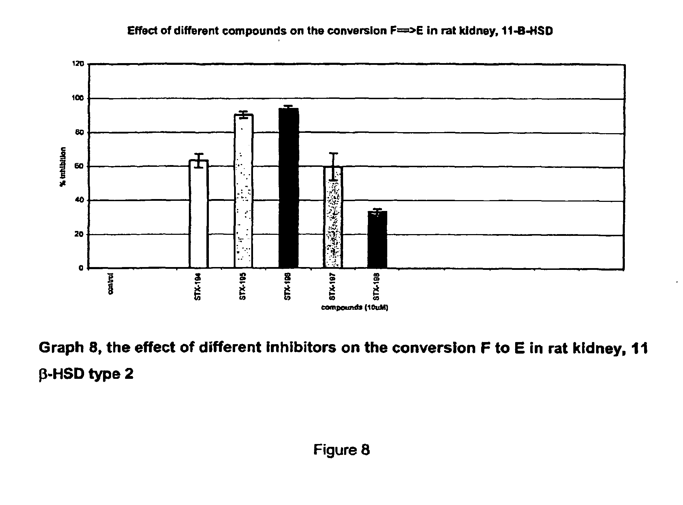

Figure 8 shows a graph;

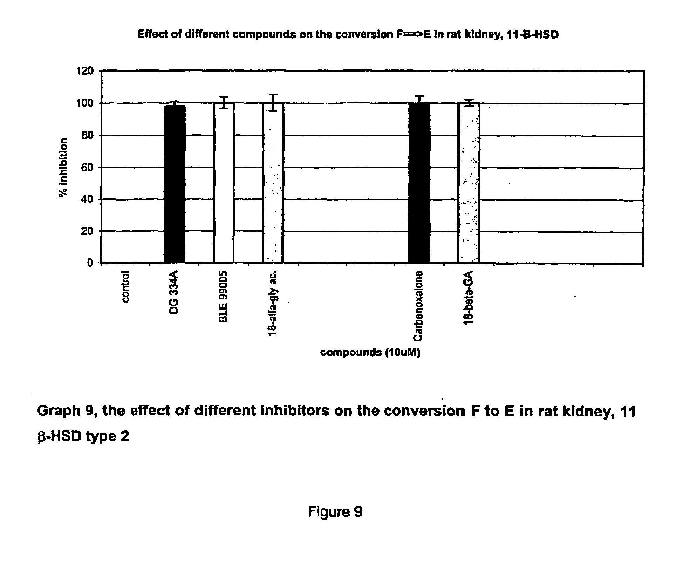

Figure 9 shows a graph;

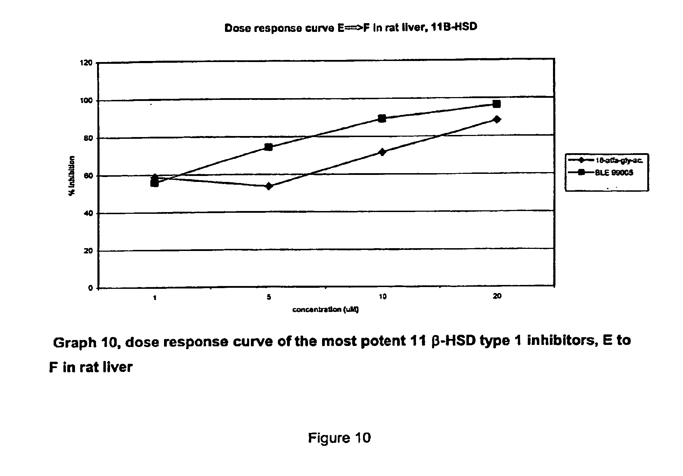

Figure 10 shows a graph; and

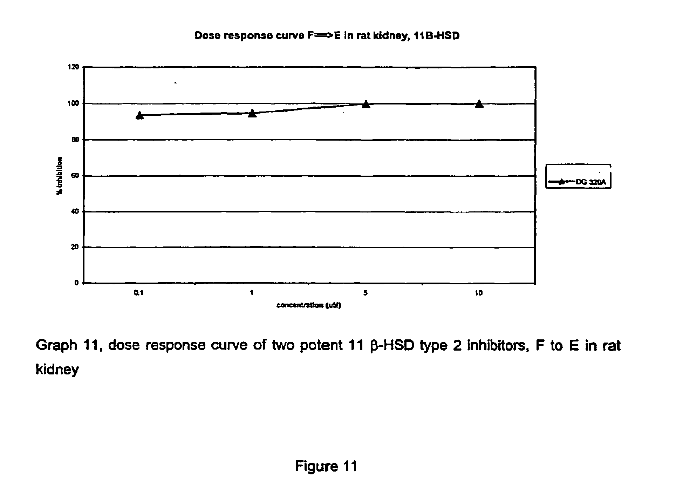

Figure 11 shows a graph.

The present invention will now be described in further detail in the following examples.

### EXAMPLES

### MATERIALS AND METHODS

### Materials

**Enzymes** - Rat livers and rat kidneys were obtained from normal Wistar rats (Harlan Olac, Bicester, Oxon,UK). Both the kidneys and livers were homogenised on ice in PBS-sucrose buffer (1g/ 10 ml) using an Ultra-Turrax. After the livers and kidneys were homogenised the homogenate was centrifuged for five minutes at 4000 rpm. The supernatant obtained was removed and stored in glass vials at -20°C. The amount of protein per µl of rat liver and kidney cytosol was determined using the Bradford method [14].

### Apparatus

- Incubator: mechanically shaken water bath, SW 20, Germany.
- Evaporator, Techne Driblock DB 3A, UK
- TLC aluminium sheets 20 x 20 cm silica gel 60 F₂₅₄, Merck, Germany.
- Scintillation vials: 20 ml polypropylene vials with caps, SARSTEDT, Germany.
- Scintillation counter: Beckman LS 6000 SC, Beckman Instruments Inc., USA.

### Solutions

- Assay medium: PBS-sucrose buffer, Dulbecco's Phosphate Buffered Saline, 1 tablet/100 ml with 0,25 M sucrose, pH 7.4 BDH Laboratory supplies, UK.
- Scintillation fluid: Ecoscint A (National Diagnostics, USA).
- Radioactive compound solutions: [1,2,6,7-³H]-cortisol (Sp. Ac. 84 Ci/mmol) NEN Germany, [4-¹⁴C]-cortisol (Sp. Ac. 53 mCi/mmol) NEN Germany.
- CrO₃ and Acetic acid (Sigma Chemical Co., UK).
- Extraction fluid: Di-ethylether, Fischer Chemicals, UK.
- Bradford Reagent solution: Coomassie Brilliant Blue G-250, 100 mg in 95% ethanol with 100 ml of phosphoric acid (85% w/v) diluted to 1 litre.

### Compounds

- Inhibitors: compounds were obtained from Sigma Chemical Co., UK or were synthesised in accordance with the synthetic routes below or as described in Appendix I.
- Cofactor NADPH and NADP, Sigma Chemical Co., UK.

### SYNTHETIC ROUTES

### Synthesis of Jones' Reagent (3)

To a solution of Chromium trioxide (2.8 g) in water (200 ml) was stirred and cooled to 0 °C. To this solution conc. H₂SO₄ (0.7 ml) was added cautiously and stirred. This solution was used for the oxidation reaction of 18β-Glycerrhetenic acid.

### 11-Deoxo-3β-benzyl-18β-glycerrhetinic acid

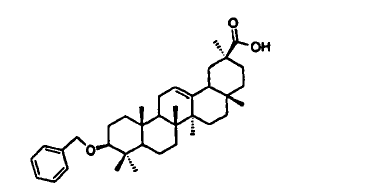

R*_{f}*: 0.87 (CHCl₃ : methanol = 9 : 1); **m.p.** : 207 - 208 °C; **MS** (FAB⁺) m/z : 547.3 [100, (M+H)⁺], 91.0 [75, (PhCH₂+H)⁺]; **MS** (FAB⁻) m/z (rel. intensity) : 545.1 [100, (M-H)⁻].

### 11-Deoxo-3α-benzyl-18β-glycerrhetinic acid

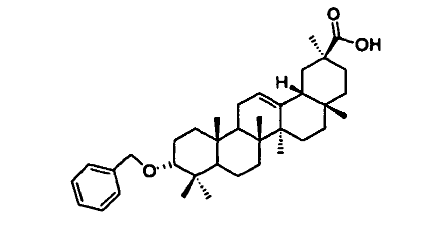

### 11-Oxo-3β-benzyloxy-18β-glycyrrhetinic acid benzyl ester (DGS01046 C) (15)

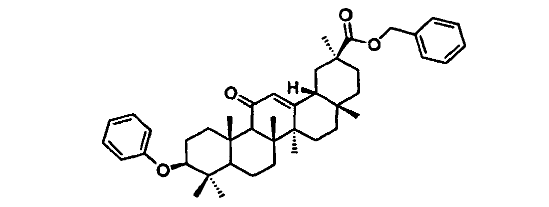

Using the procedure described for the preparation of **13**, a solution of 18β-glycerrhetinic acid (1 g; 2.12 mmol) in DMF (70 ml), NaH (255 mg; 6.37 mmol) and benzyl bromide (1 ml; 7.44 mmol) gave a white solid (2.1 g), which was purified by flash chromatography (CHCl₃/methanol gradient, 10:1 to 6:1) and the two white solids isolated (796 mg and 364 mg) were recrystallised with hot acetone to give **15** (153 mg; 43%) and **16** (139 mg; 39%) as white crystals respectively. R*_{f}*: 0.93 (CHCl₃ : methanol = 9 : 1); m.p. : 207 - 209 °C; **MS** (FAB⁺) m/z: 651.3 [100, (M+H)⁺], 91.0 [60, (PhCH₂+H)⁺].

### 11-Oxo-3β-benzyloxy-18β-glycerrhetinic acid

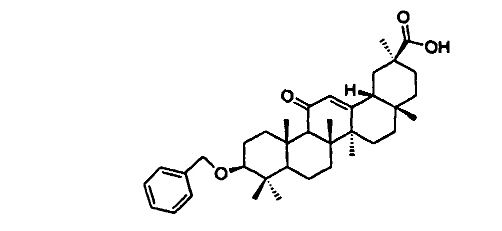

### 11-Oxo-3α-benzyloxy-18β-glycerrhetinic acid

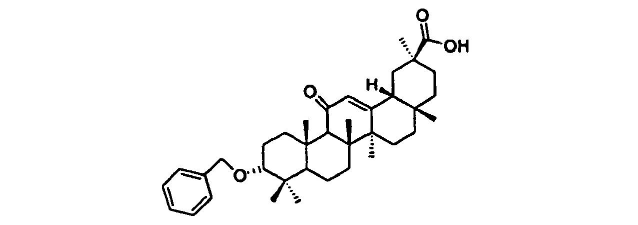

### Generation of Diazomethane (DG 336) (17)

The mini Diazald apparatus was assembled and the condenser was filled with dry ice and then acetone was added slowly until the cold finger is about one third full. In the reaction vessel, absolute ethanol (95%, 10 ml) was added to a solution of potassium hydroxide pellets (5 g) dissolved in water (8 ml). A receiver flask with a clear seal joint was attached to the condenser and the ether trap at the side arm of the condenser were cooled in an ice bath.

The reaction vessel was warmed to 65 C and the diazald (5g, 23 mmol) dissolved in diethyl ether (45 ml) was added dropwise over a period of 20 mins. The rate of distillation should be approximately equal to the rate of addition. When all of the diazald has been used up, diethyl ether (10 ml) was added slowly and continued with the distillation until the distillate is colourless. The diazomethane (17) was collected as a yellow solution in diethyl ether (700 mg; 16.6 mmol).

### 3β-Methoxy-11-oxo-18β-glycerrhetinic acid

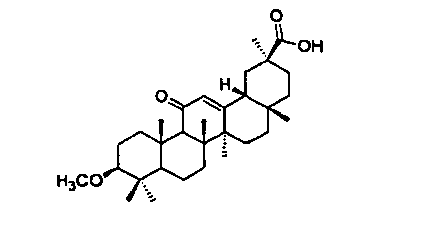

### 11-Deoxy-3β-methoxy-18β-glycyrrhetinic acid

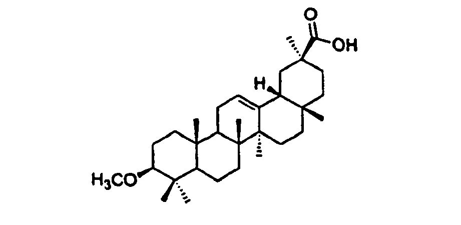

### 3β-Ethoxy-11-oxo-18β-glycerrhetinic acid

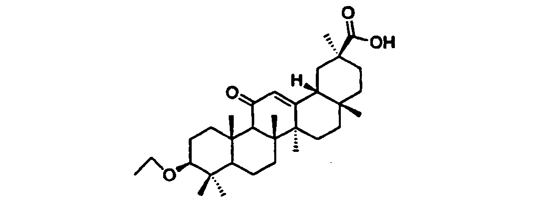

### 11-Deoxy-3β-ethoxy-18β-glycyrrhetinic acid

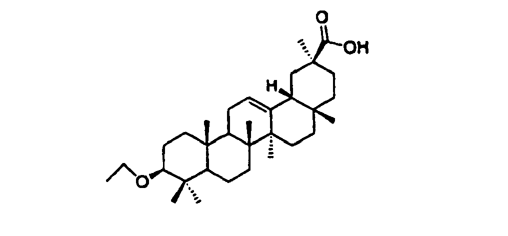

### 3β-^{tert}butoxy-11-oxo-18β-glycerrhetinic acid

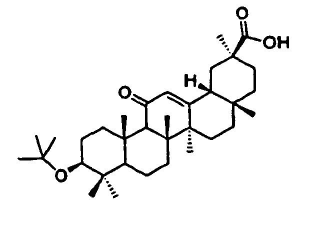

### 3α-^{tert}butoxy-11-oxo-18β-glycerrhetinic acid

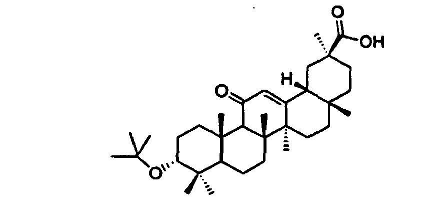

### 3β-isopropyloxy-11-oxo-18β-glycerrhetinic acid

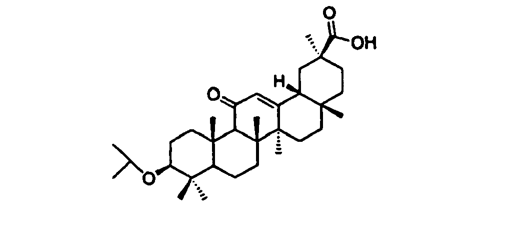

### 11-Deoxy-3β-^{iso}propyloxy-18β-glycerrhetinic acid

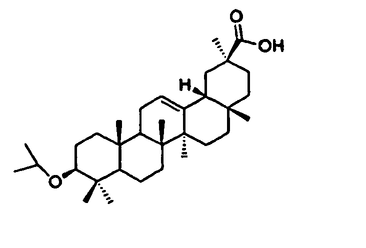

### 3β-Phenethyloxy-11-oxo-18β-glycerrhetinic acid (STX 197a)

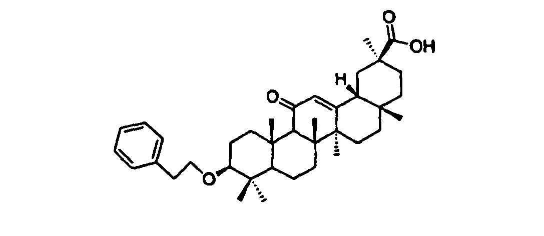

### 11-Deoxy-3β-phenethyloxy-18β-glycyrrhetinic acid

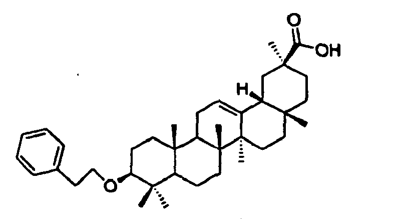

### 3β-[3-Phenylpropyl]oxy-11-oxo-18β-glycerrhetinic acid

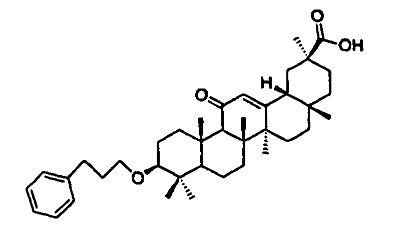

### 3α-[3-Phenylpropyl]oxy-11-oxo-18β-glycerrhetinic acid

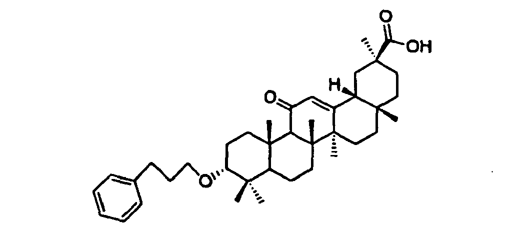

### 11-Deoxy-3β-[phenylpropyl]oxy-18β-glycyrrhetinic acid

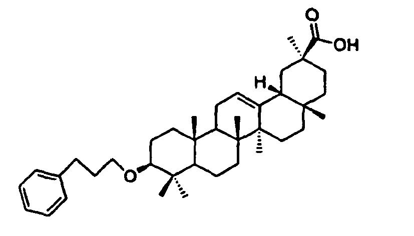

### 3β-Cyclohexyloxy-11-oxo-18β-glycerrhetinic acid

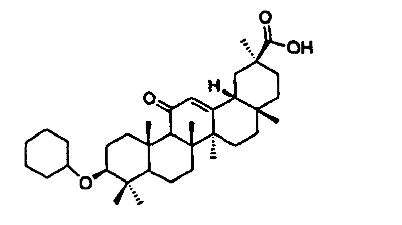

### 11-Deoxy-3β-cyclohexyloxy-18β-glycyrhetinic acid

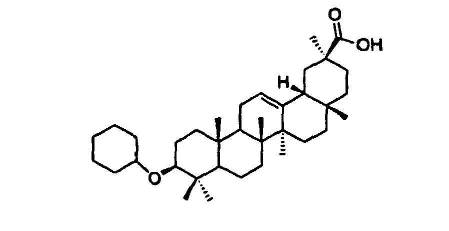

### 3β-cyclohexylmethyloxy-11-oxo-18β-glycyrrhetinic acid

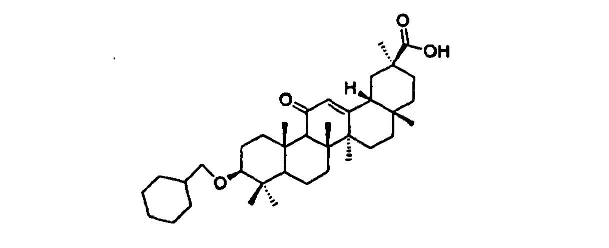

### O-Alkylation of the Methyl-ester of glycyrrhetinic acid

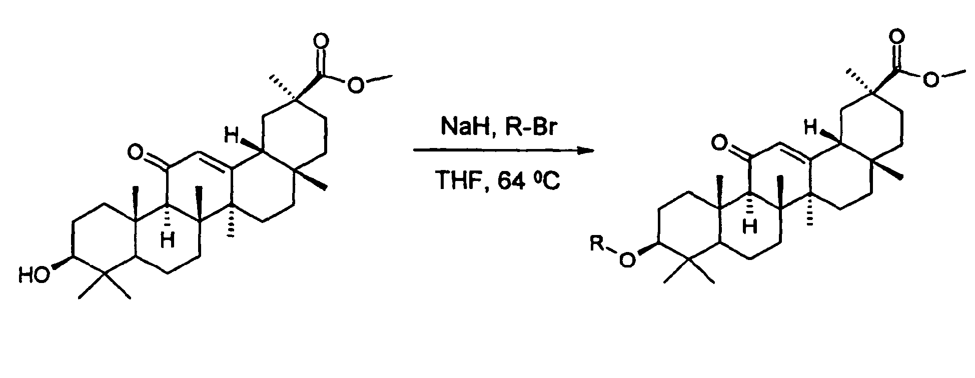

### Experimental

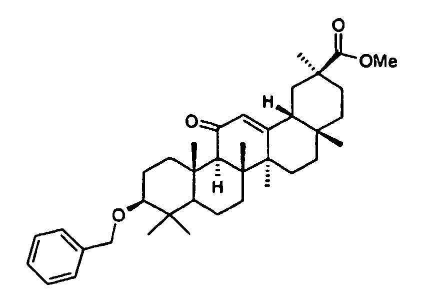

R = Benzyl STX359

To a solution of Glycyrrhetinic acid methyl ester (0.200 g, 0.000413 mol) in freshly distilled THF (5.0 ml) under nitrogen atmosphere at room temperature was added sodium hydride (0.036 mg, 0.000826 mol; 60% dispersed in mineral oil), followed by benzyl bromide (0.105 g, 0.000619 mol). The reaction mixture was stirred at room temperature for about 1 h, and refluxed at 64 °C for approximately 40 hours. Once the reaction had gone to completion (monitored by tlc), the crude mixture was cooled to room temperature and quenched by drop wise addition of water over 20 min. The aqueous layer was extracted with ethyl acetate, washed with brine, dried (MgSO₄) and concentrated under reduced pressure to give a pale yellow solid. The resulting crude product was purified by flash chromatography (EtOAc: hexane, gradient elution) to afford the benzylated product as a colourless solid (0.120 g, 51%); Diagnostic signals of ¹H NMR (CDCl₃) δ 7.35-7.32 (multiplet, 5H, phenyl), 5.66 (s, 1H, olefinic), 4.69 (d, *J* = 12.2 Hz, 1H, aromatic), 4.40 (d, *J* = 11.7 Hz, 1H, aromatic), 3.69 (s, 3H, OMe), 2.94 - 2.84 (dd, *J* = 7.4 and 11.7 Hz, 1H, CH), 2.83 - 2.80 (broad dt, 1H, CH), 2.32 (s, 1H, CH), 2.09-1.58 (multiplet, ring H), 1.56 (sharp s, H₂O), 1.41-1.38 (broad multiplet, ring H), 1.35 (s, 3H, Me), 1.32-1.19 (broad m, ring H), 1.15 (s, 3H, Me),1.14 (s, 3H, Me), 1.12 (s, 3H, Me), 1.00 (s, 3H, Me), 0.86 (s, 3H, Me), 0.80 (s, 3H, Me); HPLC: *R_{T}* = 3.57 (85%); MP = 285 °C.

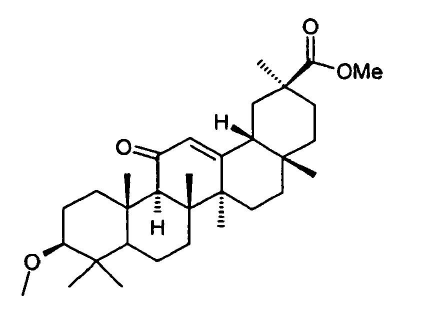

### R= Methyl HDS01028A (STX400)

This compound was synthesised using the same experimental procedure as shown for benzyl derivative (*See above*); Diagnostic signals of ¹H NMR (CDCl₃) δ 5.67 (s, 1H, olefinic), 3.69 (s, 3H, OMe), 3.36 (s, 3H, Me), 2.84 - 2.81, (broad dt, 1H, CH), 2.69 (dd, *J* = 4.7 and 11.7 Hz, 1H, CH) 2.33 (s, 1H, CH), 2.09-1.58 (multiplet, ring H), 1.56 (sharp s, H₂O), 1.41-1.38 (broad multiplet, ring H), 1.36 (s, 3H, Me), 1.32-1.19 (broad m, ring H), 1.15 (s, 3H, Me), 1.14 (s, 3H, Me), 1.12 (s, 3H, Me), 0.99 (s, 3H, Me), 0.81 (s, 3H, Me), 0.79 (s, 3H, Me); HPLC: *R_{T}* = 5.63 (> 90%)

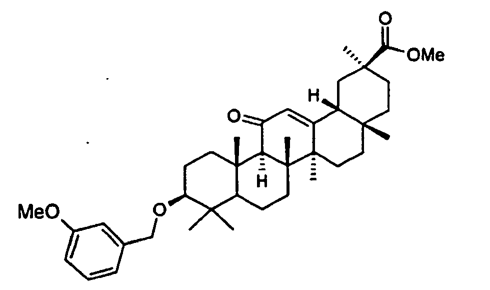

### R = 3-Methoxy benzyl HDS01028D (STX401)

*This compound was synthesised using the same experimental procedure as shown for benzyl derivative (See above); Diagnostic signals of ¹H NMR (CDCl₃) δ 7.23 (d,* J = *8 Hz, Aromatic H), 6.94 (app d, aromatic H), 6.92 (overlapping s, 1H, aromatic H) 6.82 (d,* J = *2.0 Hz, aromatic H), 6. 81 (d,* J *= 5.0 Hz, aromatic H) 5.66 (s, 1H, olefinic), 4.67 (d,* J = *12 Hz, 1H, benzylic), 4.41 (d,* J = 12.2 *Hz, 1H benzylic), 3.81 (s, 3H, OMe), 3.69 (s, 3H, OMe), 2.96 - 2.92 (dd,* J = *4.3 and 11.7 Hz, 1H, CH), 2.83* - *2. 80, (broad dt, 1H, CH), 2.33 (s, 1H, CH), 2.09-1.58 (multiplet, ring H), 1.56 (sharp s, H₂O), 1.41-1.38 (broad multiplet, ring H), 1.36 (s, 3H, Me), 1.32-1.19 (broad m*, *ring H), 1.16 (s, 3H, Me), 1.15 (s, 3H, Me), 1.13 (s, 3H, Me), 1.01 (s, 3H, Me), 0.87 (s, 3H, Me), 0.81 (s, 3H, Me); HPLC: R_{T}* = *5.03 (> 98%)*

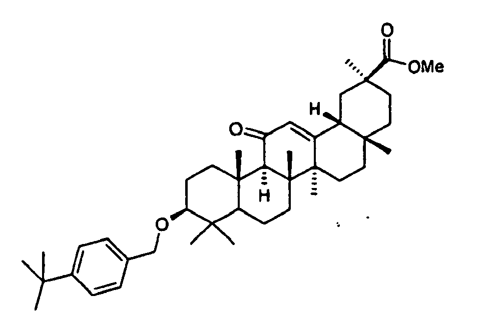

### R = para Tert-butyl benzyl STX360

This compound was synthesised using the same experimental procedure as shown for benzyl derivative (See above); Diagnostic signals of ¹H NMR (CDCl₃) δ 7.97 (d, *J* = 7.8 Hz, 2H, aromatic), 7.46 (d, *J* = 4.7 Hz, 2H, aromatic) 5.66 (s, 1H, olefinic), 4.65 (d, *J* = 11.7 Hz, 1H, benzylic H), 4.39 (d, *J* = 11.8 Hz, 1H, benzylic H), 3.69 (s, 3H, OMe), 2.96 - 2.82 (dd, *J* = 4.3 and 11.3 Hz, 1H, CH), 2.84 - 2.80 (broad dt, 1H, CH), 2.32 (s, 1H, CH), 2.09-1.58 (multiplet, ring H), 1.56 (sharp s, H₂O), 1.41-1.38 (broad multiplet, ring H), 1.36 (s, 3H, Me), 1.32-1.19 (broad m, ring H), 1.15 (s, 3H, Me),1.14 (s, 3H, Me), 1.12 (s, 3H, Me), 1.01 (s, 3H, Me), 0.86 (s, 3H, Me), 0.80 (s, 3H, Me); HPLC: *R_{T}*= 4.97 (85%)

### Synthesis of glycyrrhetinic acid amide derivatives

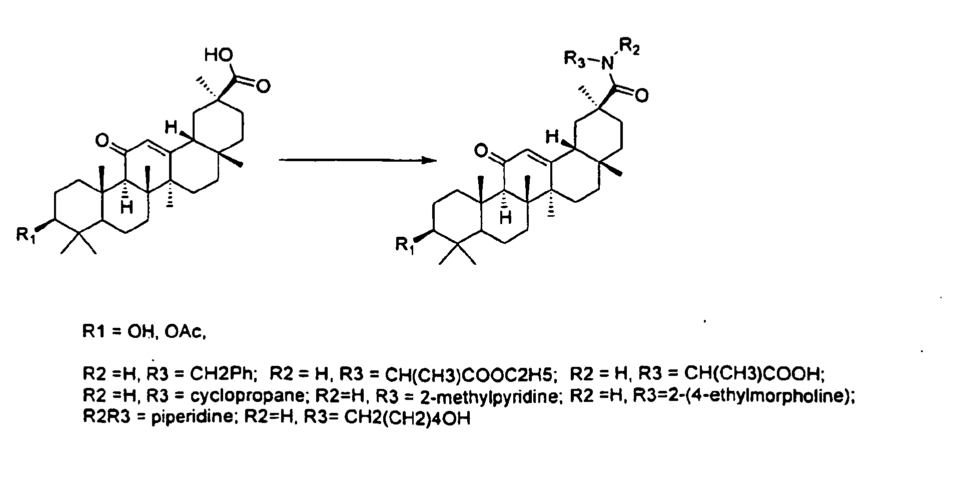

### Method A

To a solution of the acid (0.2 mmol) in chloroform ( 5 ml) was added amine ( 0.4 mmol) and EDCI (0.4 mmol). The mixture was stirred under nitrogen at room temperature for 6 to 16 hours. TLC showed the completion of the reaction. The reaction mixture was poured into water, extracted with dichloromethane. The organic phase was washed with 2% HCl and water, dried over MgSO₄. Evaporation of the solvent gave a residue, which was purified by flash chromatography. The yield was between 30 to 80%. The product was characterised by NMR, MS, Hi Res-MS, TLC and HPLC.

### Method B

To a solution of the acid (0.5 mmol) in dichloromethane (15 ml) was added amine (1.0 mmol), HOBt (0.26 mmol), EDCI (0.55 mmol), DMAP (0.55 mmol) and triethylamine (0.55 mmol). The mixture was stirred under nitrogen at room temperature for 16 to 24 hours. TLC showed the completion of the reaction. The reaction mixture was poured into water, extracted with dichloromethane. The organic phase was washed with 2% HCl and water, dried over MgSO₄. Evaporation of the solvent gave a residue, which was purified by flash chromatography. The yield was between 60 to 90%. The product was characterised by NMR, MS, Hi Res-MS, TLC and HPLC.

### 18β-glycyrrhetinic acid benzylamide STX 366 (XDS01030)

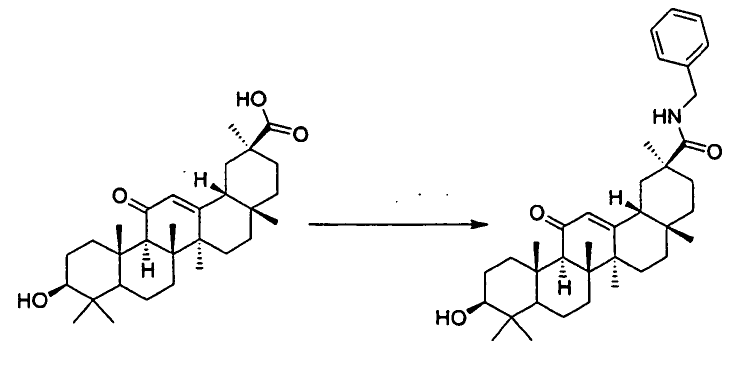

To a solution of the glycyrrhetinic acid (100 mg, 0.213 mmol) in chloroform (5 ml) was added benzylamine (0.05 ml, 0.458 mmol) and EDCl (98 mg, 0.511 mmol). The mixture was stirred under nitrogen at room temperature for 6 hours. TLC showed the completion of the reaction. The reaction mixture was poured into water, extracted with dichloromethane. The organic phase was washed with 2% HCl and water, dried over MgSO4. Evaporation of the solvent gave a residue, which was purified by flash chromatography to give off-white powder (40 mg, 33%).
TLC (5% Methanol-dichloromethane) single spot at R_{f} 0.75.
HPLC t_{R} 2.58 min, purity 98%
¹HNMR 400MHz CDCl₃ 7.26-7.37 (m, 5H, aromatic protons), 5.83 (t, 1H, NH), 5.57(s, 1H, 12-H), 4.47(m, ABX, 2H, NHCH₂Ph), 3.21 (dt, 1H, 3a-H), 2.78(dt, 1H, 18-H), 2.31 (s, 1H, 9a-H).
MS(FAB+)m/z: 560(100, M+1)

### 18β-glycyrrhetinic acid L-alanine ethyl ester amide STX369 (XDS01034)

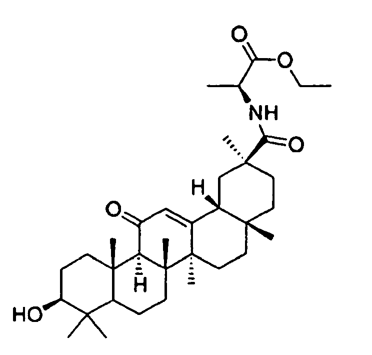

The compound was synthesised with general method B.
TLC (5% Methanol-dichloromethane) single spot at R_{f} 0.68.
HPLC t_{R} 4.21 min, purity 99%
¹HNMR 400MHz CDCl₃ 6.14 (d, 8Hz, 1H, NH), 5.76(s, 1H, 12-H), 4.60(dq, 1H, NHCH), 4.23(q, 2H, -OCH₂CH₃), 3.23(dt, 1H, 3α-H), 2.82(dt, 1H, 18-H), 2.34(s, 1H, 9α-H). MS(FAB+)m/z: 570(100, M+1)

### 18β-glycyrrhetinic acid cyclopropylamide STX370 (XDS01035)

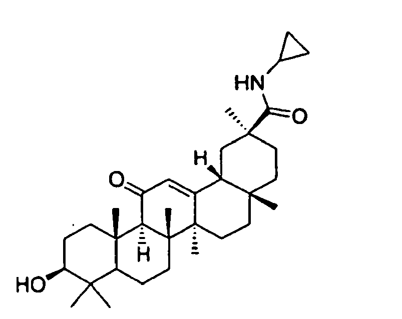

The compound was synthesised with general method B.
TLC (5% Methanol-dichloromethane) single spot at R_{f} 0.70.
HPLC t_{R} 3.89min, purity 99%
¹HNMR 400MHz CDCl₃ 5.64 (d, 2H; NH and 12-H), 3.23(dt, 1H, 3α-H), 2.82(dt, 1H, 18-H), 2.71 (m, 1H, -NHCH), 2.34(s, 1H, 9α-H).
MS(FAB+)m/z: 510(100, M+1)

### 18β-glycyrrhetinic acid (pyridin-2-ylmethyl)-amide STX371 (XDS01036)

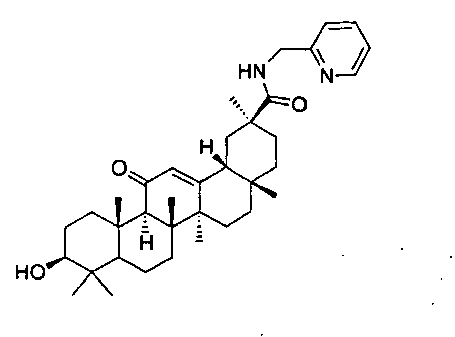

The compound was synthesised with general method B.
TLC (5% Methanol-dichloromethane) single spot at R_{f} 0.45.
HPLC t_{R} 3.69min, purity 95%
¹HNMR 400MHz CDCl₃ 8.68 (dd, 1H, 6'-H of pyridine), 7.68(td, 1H, 4'-H of pyridine), 7.36(t, 1H, NH), 7.22-7.28(m, 2H, 3',5'-H of pyridine), 5.91 (s, 1H, 12-H), 4.59(m, ABX, 2H, NHCH₂Py), 3.23(dt, 1H, 3α-H), 2.83(dt, 1H, 18-H), 2.38(s, 1H, 9α-H). MS(FAB+)m/z: 561(100, M+1)

### 18β-glycyrrhetinic acid (2-morpholin-4-yl-ethyl)-amide STX372 (XDS01037)

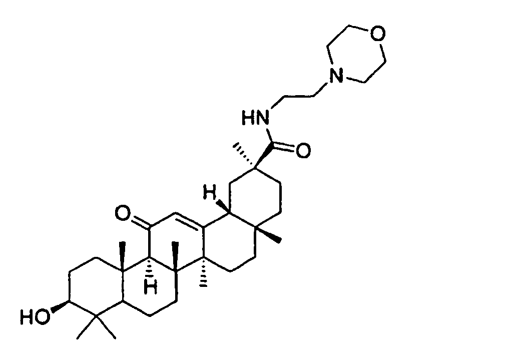

The compound was synthesised with general method B.
TLC (5% Methanol-dichloromethane) single spot at R_{f} 0.52.
HPLC t_{R} 3.92min, purity 97% ¹HNMR 400MHz CDCl₃ : 6.21(t, 1H, NH), 5.71 (s, 1H, 12-H), 3.72(m, 4H, -(CH₂)₂O of morpholine, 3.38(m, 2H, -CONHCH₂), 3.23(dt, 1H, 3α-H), 2.82(dt, 1H, 18-H), 2.20-2.40(m, 6H, -CH₂N(CH₂)₂).
MS(FAB+)m/z: 583(100, M+1)

### 18β-glycyrrhetinic acid (5-hydroxy-pentyl)-amide (XDS01039)

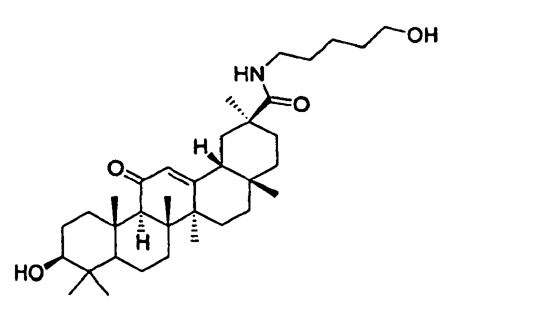

The compound was synthesised with general method B.
TLC (5% Methanol-dichloromethane) single spot at R_{f} 0.70.
¹HNMR 400MHz CDCl₃ : 5.67 (s, 1H, 12-H), 5.63(t, 1H, -NH), 3.68(dt, 2H, -NHCH₂-),
3.34(m, 2H, -CH₂OH), 3.23(dt, 1H, 3α-H), 2.80(dt, 1H, 18-H), 2.38(s, 1H, 9α-H).

### Methods

### Synthesis of radio labelled cortisone

Labelled cortisone is commercially not available. Therefore labelled cortisol (F) (³H-F and ¹⁴C-F) was oxidised at the C-11 position with CrO₃ in order to synthesize to the corresponding labelled cortisone (³H-E and ¹⁴C-E).

For this reaction F was oxidised in a 0,25% CrO₃ (w/v) dissolved in a 50% acetic-acid/distilled water (v/v) solution. The labelled F was then added to 1 ml of the CrO₃ solution, vortex mixed and put in an incubator for 20 minutes at 37°C. The aqueous reaction mixture was extracted twice with 4 ml of di-ethylether, the di-ethylether was then evaporated and the residue transferred to a TLC-plate, which was developed in the following system, chloroform : methanol 9:1 (v/v). Unlabelled cortisone (E) was also run on the TLC-plate to locate the position of the labelled steroids. After locating the spot of the labelled steroids this area is cut out from the TLC-plate and eluted with 0,5 ml of methanol.

### The amount of protein per µL of rat liver and rat kidney

The amount of protein in rat liver and rat kidney needed to be determined. The experiment was done according to the Bradford method [15]. The following method was used: first a BSA (protein) solution was prepared (1 mg/ml). Protein solutions containing 10 to 100 µg protein were pipetted into tubes and volumes adjusted with distilled water. Then 5 ml of protein reagent was added to the tubes and vortex mixed. The absorbance was measured at 595 nm after 15 minutes and before 1 hour in 3 ml cuvettes against a reagent blank. The weight of the protein was plotted against the corresponding absorbance resulting in a standard curve used to determine the protein concentration in rat liver and rat kidney cytosols.

### Assay validation - Enzyme concentration and time-dependency of 11β-HSD activity

Before carrying out 11β-HSD assays to examine the conversion E to F and F to E and the influence that different inhibitors have on these conversions the amount of rat liver homogenate and rat kidney homogenate and their incubation time need to be determined.

11 β-HSD type 1 is the enzyme responsible for the conversion E to F and this type of enzyme is present in rat liver. The substrate solution used in this assay contained 70,000 cpm/ml ³H-E in PBS-sucrose and 0.5 µM of unlabelled E and co-factor NADPH (9 mg/10 ml of substrate solution). 1 ml of the substrate solution and the different amounts of rat liver homogenate was added to all tubes.

The amount of rat liver homogenate needed for an assay was determined by incubating the substrate solution with 25, 50, 100 and 150 µl for 30, 60, 90 and 120 minutes at 37°C in a water bath with the tubes being mechanically shaken. After the incubation 50 µL of recovery solution was added, containing about 8,000 cpm/ 50 µL of ¹⁴C-F and 50 µg/50 µL of unlabelled F for visualising the spot on the TLC-plate, to correct for the losses made in the next two steps. F was then extracted from the aqueous phase with 4 ml of ether (2 x 30 sec cycle, vortex mix). The aqueous phase was then frozen using dry-ice and the organic layer was decanted and poured into smaller tubes and evaporated. 6 drops of ether were then added to the small tubes to re-dissolve the residue which was transferred to an aluminium thin layer chromatography plate (TLC-plate). The TLC-plate was developed in a TLC tank under saturated conditions. The solvent system used was chloroform: methanol 9:1 (v/v). The F spots on the TLC-plate were visualised under UV-light and cut out from the TLC-plate (R_{f}=0.45). The spots from the TLC- plate were then put into scintillation vials and 0.5 ml of methanol was added to all vials to elute the radioactivity from the TLC-plate for 5 minutes. 10 ml of Ecoscint was added to the scintillation vials and they were put into the scintillation counter to count amount of product formed.

The same procedure was used for the 11 β-HSD type 2 assay, the conversion F to E, to determine the amount of rat kidney to be used and the incubation time. Except this time the substrate solution contained ³H-F and unlabelled F and the recovery contained ¹⁴C-E and unlabelled E and cortisone has a R_{f} value of 0.65 on the TLC-plate.

### Assay procedure - The 11 β-HSD inhibitors

In these assays the influence of different inhibitors on the 11 β-HSD activity both in reductive (type 1) and oxidative (type 2) directions were assessed. In the reductive direction E is the substrate and F the product and visa versa in the case of oxidation. The method described here is for the oxidative direction.

The substrate solution contained about 50,000 cpm/ml ³H-F in PBS-sucrose and 0.5 µM F. 1ml of the substrate solution was added to each tube, the inhibitors were also added, at a 10 µM concentration, to each tube except to the "control" and "blank" tubes. 150 µL was added to all tubes except to the blanks, this was done to correct for the amount of ³H-F spontaneously formed. The tubes were incubated for 60 minutes in a mechanically shaken water bath at 37°C. The amount of kidney liver homogenate and incubation time used resulted from the enzyme- and time-dependency assay. After incubation 50 µL of recovery was added to correct for the losses made in the next steps, containing 5000 cpm/50 µL of ¹⁴C-E and 50µg/50 µL of unlabelled E (to visualise the spot on the TLC-plate). The aqueous mixture was then extracted with 4 ml of ether (2 x 30 sec cycle, vortex mix). After freezing the aqueous phase, the ether (upper) layer was decanted into smaller tubes and evaporated at 45°C until completely dry. The residue was then redissolved in 6 drops of ether and transferred to a TLC-plate. The TLC-plate was developed in chloroform : methanol (9:1 v/v) solvent system, the TLC-plate ran for about 90 minutes until the solvent front had moved about 18 cm. The position of the product E was visualised under UV-light and cut out from the TLC-plate and put into scintillation vials. Radioactivity was eluted over 5 minutes with 0.5 ml methanol. 0.5 ml of PBS-sucrose and 10 ml of Ecoscint were then added and vortex mixed before counting in the scintillation counter. Before counting the samples, two total activity vials were prepared. These contained 0.5 ml of the substrate solution, 50 µL of the recovery, 0.5 ml of methanol and 10 ml of Ecoscint. These two total activity vials were needed to determine the amount of ¹⁴C-E and ³H-F added in the beginning to make the calculations.

In case of the reductive direction, E to F ,the same method was used. Only the substrate solution containing ³H-E and unlabelled E and the recovery containing ¹⁴C-F and unlabelled F are different to the method used in the oxidative direction.

After testing all the inhibitors at 10 µM a dose-response experiment was done for the most potent 11 β-HSD type 1 and type 2 inhibitors. To look at the percentage of inhibition four different concentrations, 1, 5, 10 and 20 µM, were used. The method for both the rat liver, type 1 the reductive, and rat kidney, type 2 the oxidative, stay the same throughout the entire experiment.

### RESULTS

### The amount of protein per µL of rat liver and rat kidney

An initial experiment was carried out to determine the amount of protein in rat liver cytosol and rat kidney cytosol, to be added to each tube. Graph 1 shows the standard curve from which the amount of protein used in both experiments was calculated. The amount of protein added to each tube in the rat liver experiment was 75.5 µg (per 25 µL). In the rat kidney experiment the amount of protein added to each tube was 135.6 µg (per 150µL).

### Enzyme concentration and time-dependency of 11 β-HSD activity

In this experiment the amount of rat liver homogenate and rat kidney homogenate added to each tube and the incubation time was determined. Graph 2 shows the enzyme concentration and time-dependency course of the rat liver experiment E to F, 11 β-HSD type 1 activity. Graph 3 shows the enzyme concentration and time-dependency course F to E, 11 β-HSD type 2 activity. After drawing the graphs the optimal amount of rat liver cytosol and rat kidney cytosol and both their incubation times were selected. One important rule when selecting both variables, to select an amount of rat liver and rat kidney and incubation time on a linear part of the graph. This is done to avoid fluctuations in enzyme activity. The amount of rat liver cytosol selected was 25 µL and 90 minutes of incubation time, the amount of rat kidney cytosol selected was 150 µL and 60 minutes of incubation time.

### The 11 β-HSD inhibitors

In this experiment the influence of different inhibitors on the conversion E to F and F to E was determined. The reason why inhibition in both directions was examined was to make a comparison between the inhibitors and which type of 11 β-HSD they inhibit more. Thirty-two compounds were screened for their ability to inhibit 11 β-HSD type 1 (E to F) and type 2 (F to E). All the inhibitors were initially tested at a 10 µM concentration. Their inhibitory effects on the conversion E to F are shown in graphs 4-6 and their inhibitory effects on the conversion F to E are shown in graphs 7-9. The percent of inhibition was calculated as the percentage of decrease in radio labelled ³H-E and ³H-F of product formed, compared with the control activity (the tubes without an inhibitor in it). All the results calculated are means, n=2.

The most potent inhibitors where screened at four different concentrations, 1, 5, 10 and 20 µM. to further determine the inhibitory effect of these compounds. The dose response curve of the most potent 11 β-HSD type 1 inhibitors are shown in graph 10. Graph 11 shows a dose response of three potent 11 β-HSD type 2 inhibitors.

In table 1, the structures of the inhibitors from the glycyrrhetinic acid derivative group are drawn and their percent of inhibition on the conversion E to F and F to E is shown. The inhibition of 11 β-HSD type 1 by the glycyrrhetinic acid derivatives ranged from 22% for BLE99006 to 87% for BLE99005.

The inhibition of 11 β-HSD type 2 was also examined all inhibitors were divided into the same groups. For the glycyrrhetinic acid derivatives the inhibition ranged from 33% for STX-198 to 100% for BLE 99005, DG 320A, 18α-glycyrr. Acid, 18β-glycyrr. Acid and carbenoxalone.

**Table 1 - The Inhibitory Effect of Glycyrrhetinic Acid Derivatives**

| COMPOUND (10 µM) | | | % INHIBITION | |
|---|---|---|---|---|
| CODE | NAME | STRUCTURE | E→F ± SD | F→E ± SD |
| DG 381A (STX122) | | 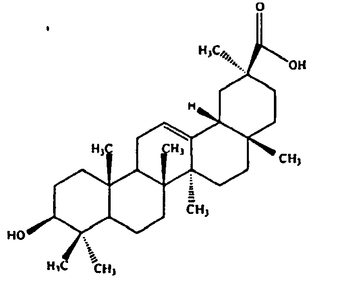 | 92.67 ± 1.23 | 109.03 ± 1.64 |
| BLE99005 | | 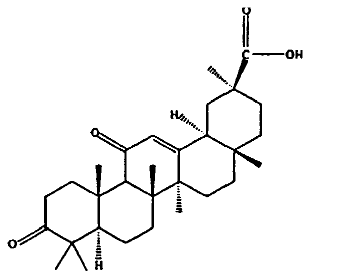 | 86,9 0,882 | ± 100 ± 3,566 |
| STX353 18-α-GA | 3β-Hydroxy-11-oxo-18α,20β-olean-12-en-29-oic acid | 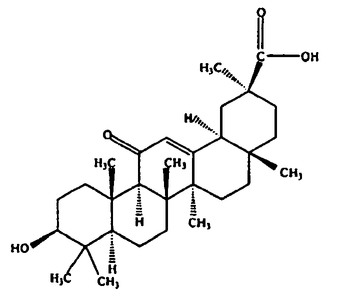 | 89.05 ± 1.49 | 100.47 ± 0.42 |
| | 18α-Glycyrrhetinic acid | | | |
| BLE99006 | 3-Oxo-oleanoic acid | 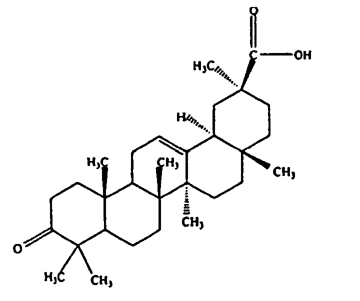 | 22,2 ± 0,354 | 45,6 ± 11,030 |
| | Carbenoxalone (disodium salt) | 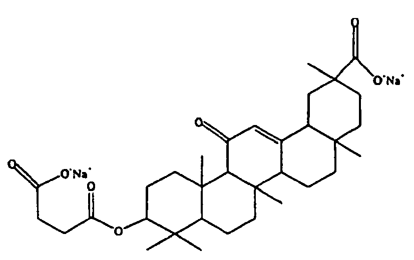 | 52,2 ± 4,799 | 100 ± 4,161 |
| 18-β-GA STX352 | 3β-Hydroxy-11-oxo-18β,20β -olean-12-en-29-oic acid | 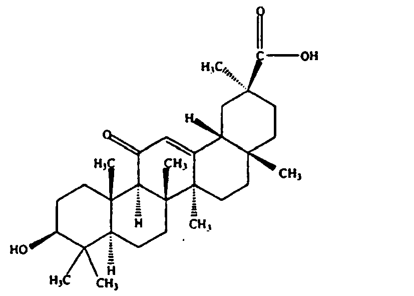 | 85.17 3.69 | 101.01 ± 0.91 |
| | 18β-Glycyrrhetinic acid | | | |
| STX194 (DGS01082B) | | 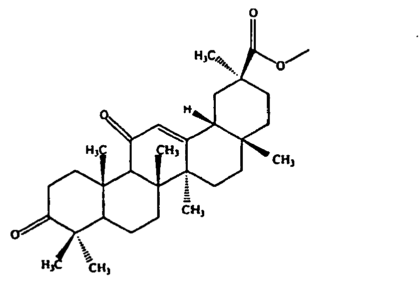 | 65.79 ± 5.69 | 75.05 ± 2.93 |
| STX195 DG 334B STX121 DG 334A (DGS01056A) | | 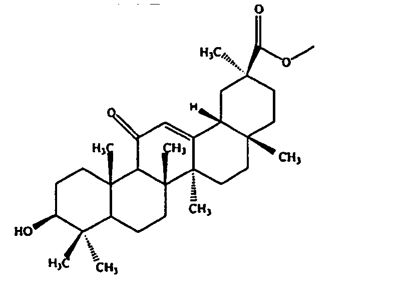 | 85.43 ± 2.29 | 105.21 ± 1.55 |
| STX196 (DGS01058A) | | 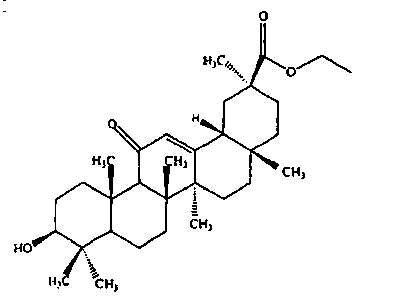 | 80.65 ± 2.14 | 97.52 ± 1.37 |
| STX195a (DGS01056A) | | 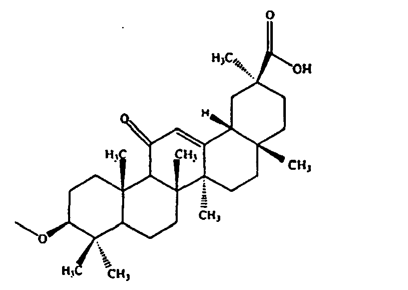 | 53,0 ± 1,023 | 90.3 ± 1,979 |
| STX196a (DGS01058A) | | 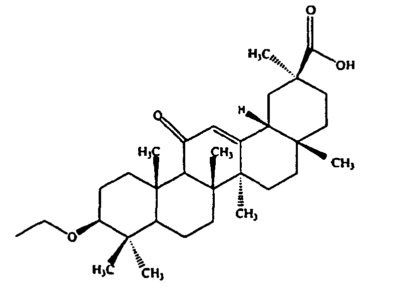 | 55,0 ± 0,022 | 93,9 ± 1,767 |
| STX197 (DGS01072A) | | 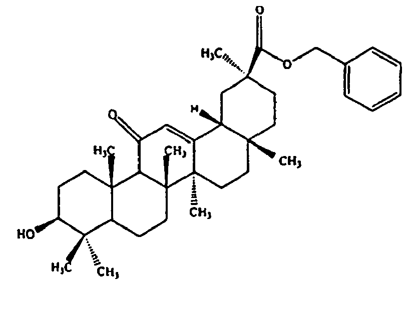 | 53,0 ± 0,935 | 59,7 ± 7,990 |
| STX198 (DGS01070A) | | 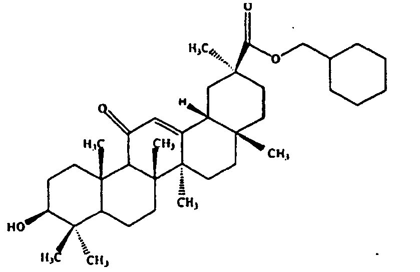 | 52.3 ± 1,253 | 33,1 ± 1,838 |
| STX 296 | | 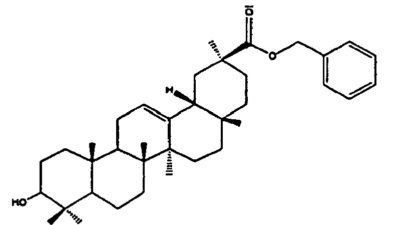 | 37.74 ± 8.85 | 33.76 ± 15.82 |
| STX 297 | | 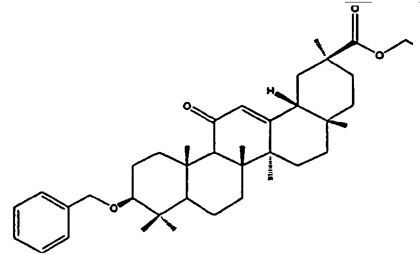 | 34.34 ± 8.26 | 58.70 ± 10.41 |
| STX 298*** | | 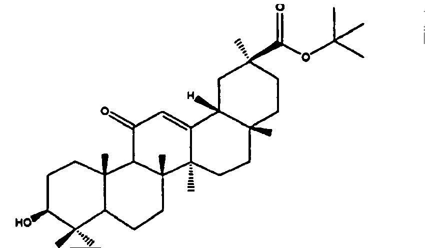 | 50.59 ± 4.43 | 22.59 ± 12.90 |
| STX 299 | | 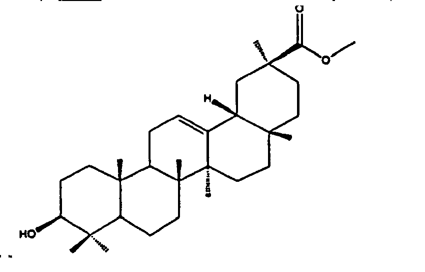 | 20.24 ± 1.89 | 19.54 ± 1.66 |
| STX 347 DG 320A | | 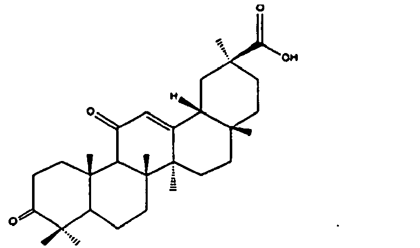 | 89.37 ± 0.10 | 102.26 ± 1.28 |
| STX 348 | | 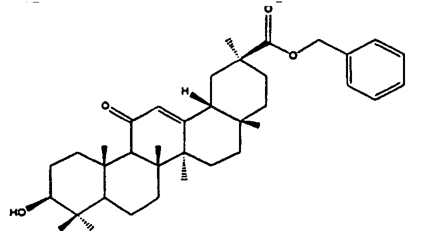 | 63.39 ± 1.45 | 94.77 ± 0.17 |
| STX 349 | | 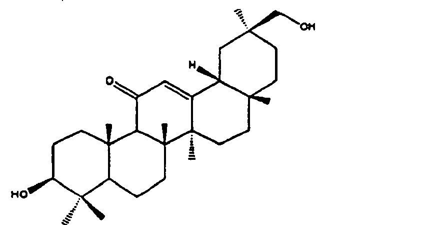 | 89.68 ± 4.90 | 100.05 ± 0.49 |
| STX 350 | | 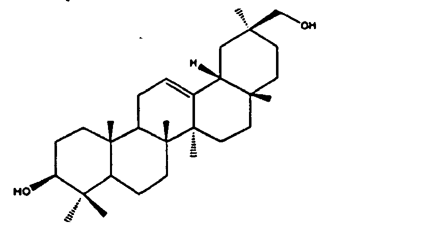 | 13.41 ± 9.63 | 60.34 ± 3.99 |
| STX 351 | | 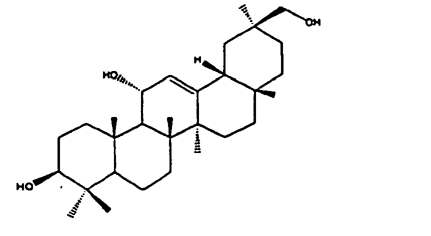 | 70.02 ± 6.39 | 94.41 ± 0.63 |
| STX 354 | | 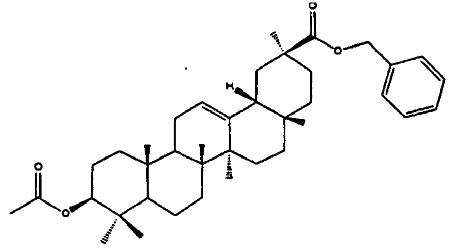 | -1.18 ± 9.22 | 13.60 ± 1.42 |
| STX 359 | | 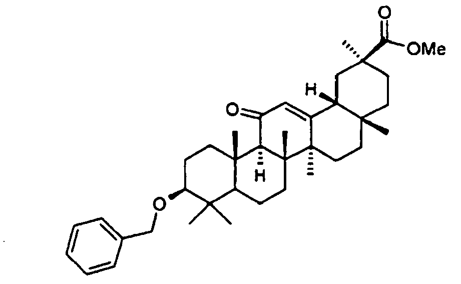 | 17.8 ± 2.5 | 17.0 ± 3.5 |
| STX 360 | | 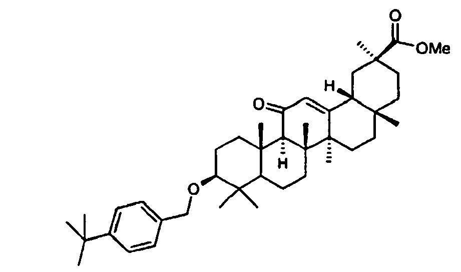 | 14.7 ± 2.4 | 26.4 ± 11.6 |
| STX 366 | | 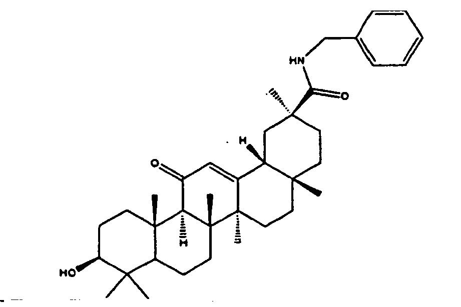 | 85.25 ± 1.09 | 74.19 ± 10.34 |
| STX 367 | | 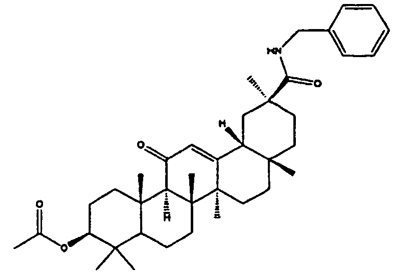 | 36.17 ± 3.69 | 27.26 ± 2.99 |
| STX 369 | | 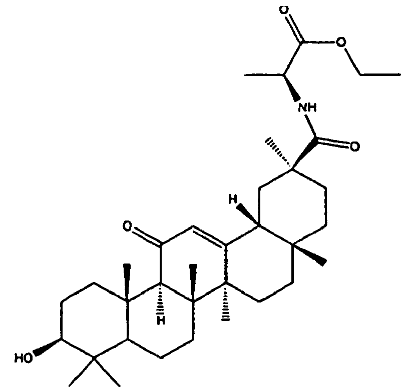 | 24.91 ± 2.51 | 35.38 ± 12.60 |
| STX 370 | | 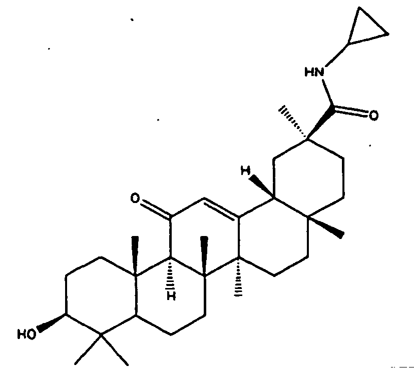 | 89.63 ± 1.18 | 102.52 ± 0.22 |
| STX 371 | | 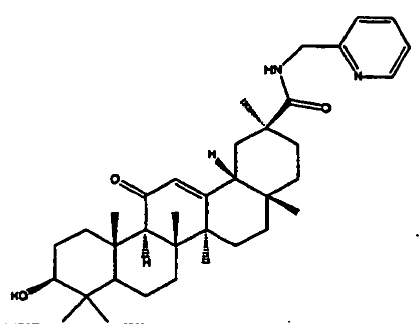 | 57.15 ± 5.60 | 92.97 ± 2.75 |
| STX 372 | | 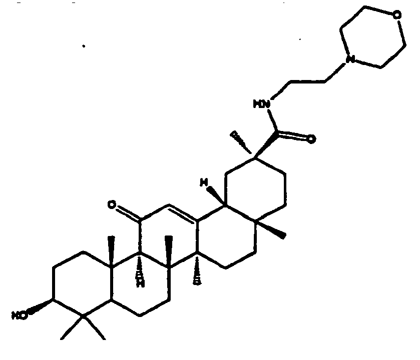 | 19.35 ± 7.81 | 65.85 ± 20.91 |

### APPENDIX I

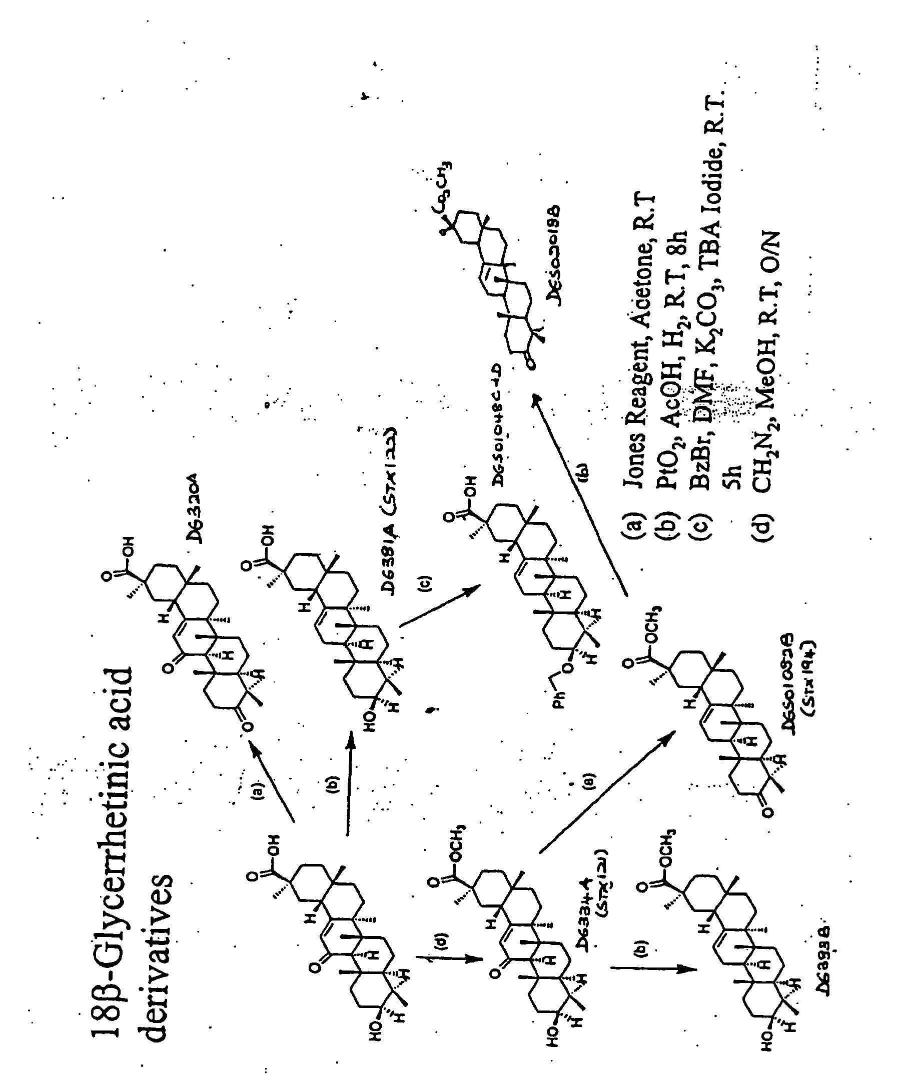

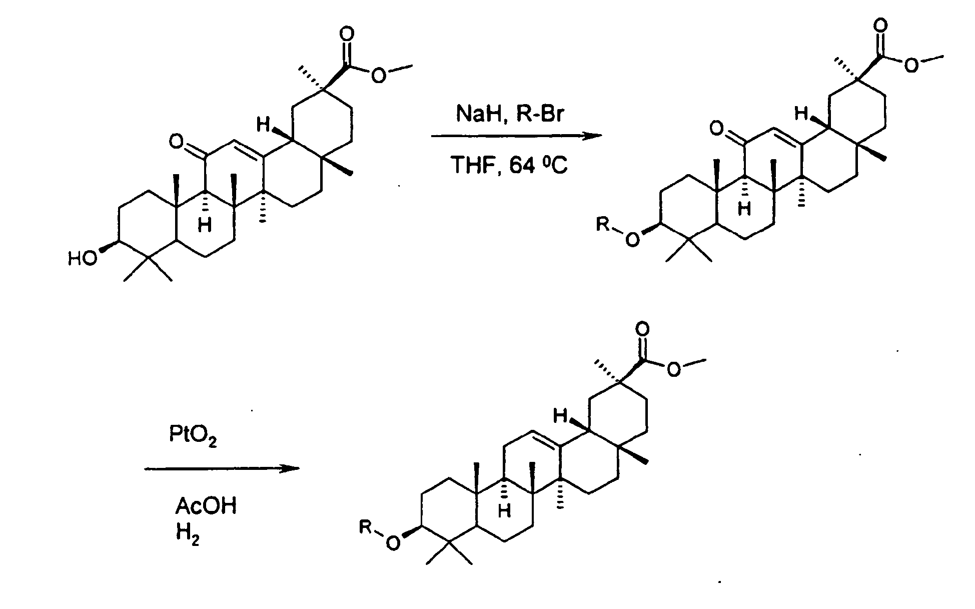

R = CH₃
Et
^{t}Bu
ⁱPr
CH₂Ph
(CH₂)₂Ph (CH₂)₃Ph
Cyclohexyl
CH₂Cyclohexyl

### REFERENCES

1. Hammond, GH (1990) : Molecular properties of corticosteroid binding globulin and sex-steroid binding proteins. Endocr. Rev. 11, 65- 79.
2. Gomez-Sanchez EP,Gomex-Sanchez CE (1997): First there was one, then two ..why not more 11 β-Hydroxysteroid Dehydrogenases? Endocrinology vol. 138, 12.
3. Krozowski ZS, Funder JW (1983): Renal mineralocorticosterone receptors and hippocampal corticosterone binding species have identical intrinsic steroid specificity . Proc. Natl. Sci. USA 80: 6056-60
4. Ulick S, Levine LS, Gunczler P, Zanconato G, Rarnirez LC, Rauh W, Rosler A, Bradlow HL, Mew MI (1979): A syndrome of apparent mineralocorticoid excess associated with defects in the peripheral metabolism of cortisol. J. Clin. Endo. And Metab. 49: 757-64.
5. Edwards CRW, Stewart PM, Burt D, Brett L, McIntyre MA, Sutanto WS, Kloet ER, Monder C (1998): Localisation of 11 ±-HSD-tissue specific protector of the mineralocorticoid receptor. Lancet 2: 986-989.
6. Moore CCD, Melloh SH, Murai /, Siiteri PK, Miller WL (1993): Structure and function of the hepatic form of 11 β-HSD in the squirrel monkey, an animal model of glucocorticoid resistance. Endocrinology 133: 368-375.
7. Kotelevtsev YV, larnieson PM, Best R, Stewart F, Edwards CRW, Seckl JR, Mullins II (1996): Inactivation of 11 β-HSD type 1 by gene targeting in mice. Endocrinology Res. 22: 791-792.
8. Ricketts ML, Verhaeg JM, Bujalska I, Howie AJ, Rainey WE, Stewart PM (1998): Immunohistochemicallocalisation of type 1 11β-HSD in human tissues. /. Clin. Endoc. Metab. 83: 1325-35.
9. Stewart PM, Sheppard MC (1992): Novel aspects ofhorrnone action: intracellular ligand supply and its control by a series of tissue specific enzymes. Molecular and Cellular Endocrinology 83: C13-C18.
10. Seckl JR, Chapman KE (1997): The 11 β-HSD system, a determinant of glucocorticoid and mineralocorticoid action. Medical and physiological aspects. European I. Biochem. 249: 361-364.
11. Maser E (1998): 11 β-HSD responsible for carbonyl reduction of the tobacco-specific nitrosoamine in mouse lung microsomes. Cancer Res. 58: 2996-3003.
12. Walker BR, Stewart PM, Shackleton C H L, Padfield PL, Edwards CRW (1993): Deficient inactivation of cortisol by 11 β-HSD in essential hypertension. Clin. Endocr. 38: 221-227.
13. Daynes RA, Araneo BA (1998) : Contrasting effects of glucocorticoids on the capacity of T -cells to produce the growth factors interleukin-2 and interleukin-4. Eur. J. Immunol. 19: 2319-2324.
14. Bradford MM (1976): A rapid and sensitive method for the quantitation of microgram quantities of protein utilizing the principle of protein-dye binding. Anal. Biochem. 72: 248-254.
15. Diederich S, Grossmann C, Hanke B, Quinkler M, Herrrnann M, Bahr V, Oelkers W (2000): In the search for specific inhibitors ofhuman 11 β-HSD: chenodeoxycholic acid selectively inhibits 11 β-HSD type 1. Europ. J. Endocrin. 142: 200-207.

## Claims

1. A compound of formula I or a salt thereof 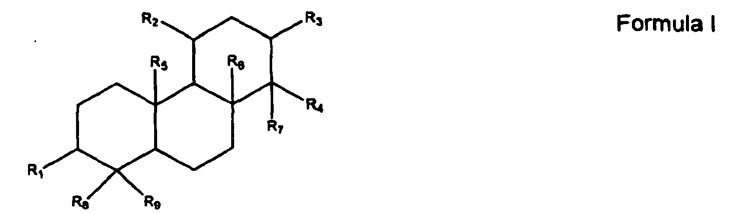 wherein R1 is O-CH₂-CH₂-Ph, O-CH₂-cyclohexyl, OH, O-alkyl, or O-aryl; and R2 is selected from H, =O, OH, hydrocarbyl, oxyhydrocarbyl, and halo; R5 to R9 are independently selected from H and hydrocarbyl
R3 and R4 together represent a group of formula II 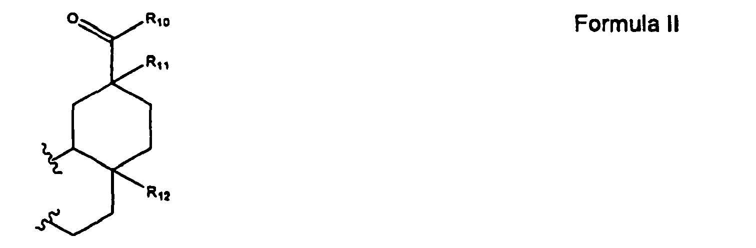 wherein R10 is selected from OH, hydrocarbyl, -NR₁₈R₁₉and O-hydrocarbyl, R11 and R12 are independently selected from H and hydrocarbyl,
wherein when R1 is OH, R10 is -NR₁₈R₁₉;
wherein R₁₈ and R₁₉ are selected from H, CH₂Ph, CH(CH₃)COOC₂H₅, CH(CH₃)COOH, cyclopropane, 2-methylpyridine, 2-(4-ethylmorpholine) and CH₂(CH₂)₄OH;
and wherein the compound may contain one or more further degrees of unsaturation.

2. A compound according to claim 1 wherein R1 is O-Me or O-Et.

3. A compound according to claim 1 or 2 wherein R2 is selected from H, =O, O-alkylaryl, and halo.

4. A compound according to any preceding claim wherein R10 is selected from OH and OMe.

5. A compound according to any preceding claim wherein R11 is Me.

6. A compound according to any preceding claim wherein R12 is Me.

7. A compound according to any preceding claim wherein R5 is Me.

8. A compound according to any preceding claim wherein R6 is Me or H.

9. A compound according to any preceding claim wherein R7 is Me.

10. A compound according to any preceding claim wherein R8 is H or Me.

11. A compound according to any preceding claim wherein R9 is H or Me

12. A pharmaceutical composition comprising the compound according to any preceding claim admixed with a pharmaceutically acceptable carrier, diluent, excipient or adjuvant.

13. A compound according to any one of claims 1 to 11 for use in medicine.

14. Use of a compound as defined in any one of claims 1 to 11 in the manufacture of a medicament for use in the therapy of a condition or disease associated with 11β-hydroxysteroid dehydrogenase.

15. Use of a compound as defined in any one of claims 1 to 11 in the manufacture of a medicament for use in the therapy of a condition or disease associated with adverse 11β-hydroxysteroid dehydrogenase levels.

## Patentansprüche

1. Verbindung der Formel I oder ein Salz davon 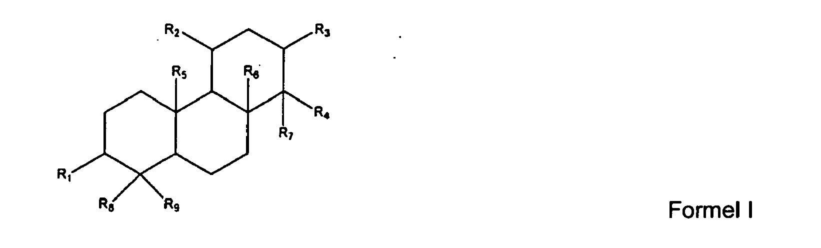 wobei R1 O-CH₂-CH₂-Ph, O-CH₂-Cyclohexyl, OH, O-Alkyl oder O-Aryl ist,
und R2 ausgewählt ist unter H, =O, OH, Kohlenwasserstoffrest, Oxykohlenwasserstoffrest und Halogen,
R5 bis R9 unabhängig voneinander ausgewählt sind unter H und Kohlenwasserstoffrest,
R3 und R4 gemeinsam eine Gruppe der Formel 11 repräsentieren 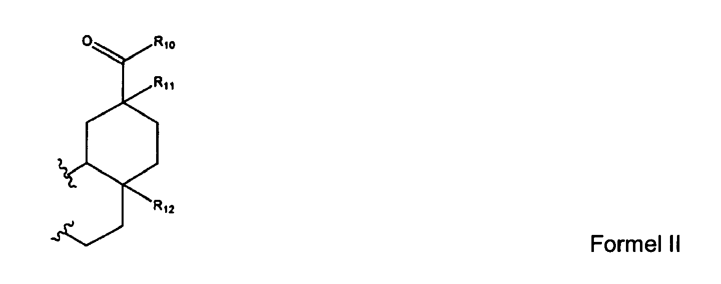 wobei R10 ausgewählt ist unter OH, Kohlenwasserstoffrest, -NR₁₈R₁₉ und O-Kohlenwasserstoffrest, R11 und R12 unabhängig ausgewählt sind unter H und Kohlenwasserstoffrest,
wobei R10 -NR₁₈R₁₉ ist, wenn R1 OH ist,
wobei R₁₈ und R₁₉ ausgewählt sind unter H, CH₂Ph, CH(CH₃)COOC₂H₅, CH(CH₃)COOH, Cyclopropan, 2-Methylpyridin, 2-(4-Ethylmorpholin) und CH₂(CH₂)₄OH,
und wobei die Verbindung einen oder mehrere weitere Grade der Ungesättigtheit enthalten kann.

2. Verbindung nach Anspruch 1, wobei R1 O-Me oder O-Et ist.

3. Verbindung nach Anspruch 1 oder 2, wobei R2 ausgewählt ist unter H, =O, O-Alkylaryl und Halogen.

4. Verbindung nach einem der vorangehenden Ansprüche, wobei R10 ausgewählt ist unter OH und OMe.

5. Verbindung nach einem der vorangehenden Ansprüche, wobei R11 Me ist.

6. Verbindung nach einem der vorangehenden Ansprüche, wobei R12 Me ist.

7. Verbindung nach einem der vorangehenden Ansprüche, wobei R5 Me ist.

8. Verbindung nach einem der vorangehenden Ansprüche, wobei R6 Me oder H ist.

9. Verbindung nach einem der vorangehenden Ansprüche, wobei R7 Me ist.

10. Verbindung nach einem der vorangehenden Ansprüche, wobei R8 H oder Me ist.

11. Verbindung nach einem der vorangehenden Ansprüche, wobei R9 H oder Me ist.

12. Pharmazeutische Zusammensetzung umfassend die Verbindung nach einem der vorangehenden Ansprüche im Gemisch mit einem pharmazeutisch verträglichen Träger, Verdünnungsmittel, Hilfsstoff oder Adjuvanz.

13. Verbindung nach einem der Ansprüche 1 bis 11 für die Verwendung in der Medizin.

14. Verwendung einer Verbindung, wie sie in einem der Ansprüche 1 bis 11 definiert ist, bei der Herstellung eines Arzneimittels für die Verwendung bei der Therapie eines Zustandes oder einer Erkrankung, welche(r) mit 11β-Hydroxysteroiddehydrogenase assoziiert ist.

15. Verwendung einer Verbindung, wie sie in einem der Ansprüche 1 bis 11 definiert ist, bei der Herstellung eines Arzneimittels für die Verwendung bei der Therapie eines Zustandes oder einer Erkrankung, welche(r) mit nachteiligen Niveaus an 11β-Hydroxysteroiddehydrogenase assoziiert ist.

## Revendications

1. Composé de formule I ou un sel de ce composé 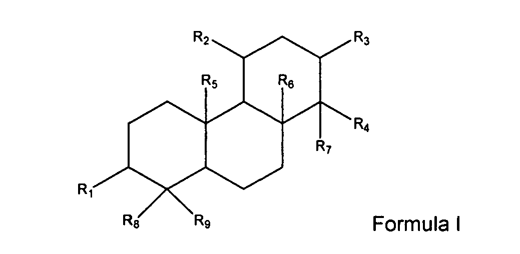 dans lequel R1 est O-CH₂-CH₂-Ph, O-CH₂-cyclohexyl, OH, O-alkyl, ou O-aryl ; et R2 est choisi parmi H, =O, OH, hydrocarbyl, oxyhydrocarbyl, et halo ;
R5 à R9 sont indépendamment choisis parmi H et hydrocarbyl ;
R3 et R4 ensemble représentent un group de formule II 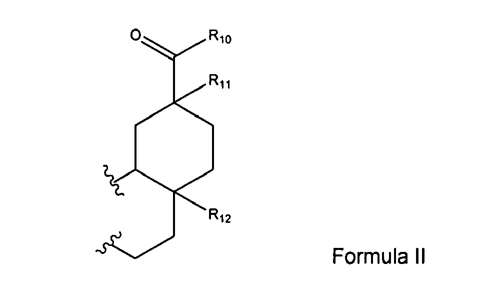 dans lequel R10 est choisi parmi OH, hydrocarbyl, -N-R₁₈R₁₉ et O-hydrocarbyl ; R11 et R12 sont indépendamment choisis parmi H et hydrocarbyl ;
lorsque R1 est OH, R10 est -N-R₁₈R₁₉ ;
lorsque R₁₈ et R₁₉ sont choisis parmi H, -CH₂-Ph, CH(CH₃)COOCH₂H₅, CH(CH₃)COOH, cyclopropane, 2-méthylpyridine, 2-(4-éthylmorpholine) et CH₂(CH₂)₄OH ;
et dans lequel le composé peut contenir un ou plusieurs degrés d'insaturation supplémentaires.

2. Composé selon la revendication 1 dans lequel R1 est O-Me ou O-Et.

3. Composé selon la revendication 1 ou 2 dans lequel R2 est choisi parmi H, =O, O- alkylaryl et halo.

4. Composé selon l'une des revendications précédentes dans lequel R10 est choisi parmi OH et OMe.

5. Composé selon l'une des revendications précédentes dans lequel R11 est Me.

6. Composé selon l'une des revendications précédentes dans lequel R12 est Me.

7. Composé selon l'une des revendications précédentes dans lequel R5 est Me.

8. Composé selon l'une des revendications précédentes dans lequel R6 est Me ou H.

9. Composé selon l'une des revendications précédentes dans lequel R7 est Me.

10. Composé selon l'une des revendications précédentes dans lequel R8 est H ou Me.

11. Composé selon l'une des revendications précédentes dans lequel R9 est H ou Me.

12. Composition pharmaceutique comprenant un composé selon l'une des revendications précédentes, en mélange avec un support, diluent, excipient ou adjuvant pharmaceutiquement acceptable.

13. Composé selon l'une des revendications 1 à 11, pour son utilisation comme médicament.

14. Utilisation d'un composé tel que défini à l'une des revendications 1 à 11 pour la préparation d'un médicament pour une utilisation dans le traitement de condition ou maladie associée à la 11 β-hydroxystéroide déhydrogénase.

15. Utilisation d'un composé tel que défini à l'une des revendications 1 à 11 pour la préparation d'un médicament pour une utilisation dans le traitement de condition ou maladie associée à des taux indésirables de 11 β-hydroxystéroide déhydrogénase.
